(19) Europäisches Patentamt
European Patent Office
Office européen des brevets

(11) EP 3 107 391 B1

# (12) EUROPEAN PATENT SPECIFICATION

(45) Date of publication and mention of the grant of the patent:
**28.03.2018 Bulletin 2018/13**

(21) Application number: **15706403.1**

(22) Date of filing: **17.02.2015**

(51) Int Cl.:
***A01N 43/42*** (2006.01) ***A01N 43/56*** (2006.01)
***A01P 7/00*** (2006.01) ***A01P 21/00*** (2006.01)

(86) International application number:
**PCT/EP2015/053249**

(87) International publication number:
**WO 2015/124542 (27.08.2015 Gazette 2015/34)**

## (54) FUNGICIDAL COMPOSITIONS OF PYRAZOLECARBOXYLIC ACID ALKOXYAMIDES

FUNGIZIDZUSAMMENSETZUNGEN VON PYRAZOLKARBOXYLSÄURENALKOXYAMIDEN

COMPOSITIONS FONGICIDES D'ACIDE PYRAZOLECARBOXYLIQUE ALKOXYAMIDES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.02.2014 EP 14155785**

(43) Date of publication of application:
**28.12.2016 Bulletin 2016/52**

(73) Proprietor: **Bayer CropScience Aktiengesellschaft**
**40789 Monheim am Rhein (DE)**

(72) Inventors:
- **HOFFMANN, Sebastian**
  **41470 Neuss (DE)**
- **WACHENDORFF-NEUMANN, Ulrike**
  **56566 Neuwied (DE)**
- **COQUERON, Pierre-Yves**
  **F-69009 Lyon (FR)**
- **CRISTAU, Pierre**
  **F-69009 Lyon (FR)**
- **DESBORDES, Philippe**
  **F-69006 Lyon (FR)**
- **DAHMEN, Peter**
  **41470 Neuss (DE)**

(74) Representative: **BIP Patents**
**c/o Bayer Intellectual Property GmbH**
**Alfred-Nobel-Straße 10**
**40789 Monheim am Rhein (DE)**

(56) References cited:
**EP-A1- 1 736 471** **EP-A1- 2 314 583**
**EP-A1- 2 621 277** **US-A1- 2012 289 702**

- **"Case 72913 Biological data", , 24 June 2013 (2013-06-24), XP055114898, Retrieved from the Internet: URL:https://register.epo.org/application?showPdfPage=1&documentId=EUSQBWCM6310FI4&ap pnumber=EP11776380 [retrieved on 2014-04-23]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 107 391 B1

## Description

The present invention relates to novel compositions, to a process for preparing these compositions and to the use thereof as biologically active compositions, especially for the control of harmful microorganisms in plants.

It is already known that certain pyrazolecarboxylic acid alkoxyamides can be used as fungicides (see WO-A 2010/063700).

The production of these compounds is described in WO-A 2010/063700 , in WO-A 2013127764 and in WO-A 2013/167651.

Moreover, it is known that these compounds can be mixed with different safener compounds (WO-A 2012/021250), with different quinazoline compounds (WO-A 2012/069652), with different pyridylamidine compounds (WO-A 2012/146125), with different isoxazole compounds (WO-A 2013/007550 and WO-A 2013/011010). Some combinations of pyrazolecarboxylic acid alkoxyamides are also described in WO-A 2012/041874 but no experimental data supporting these combinations are disclosed. WO-A 2014/016279 discloses ternary mixtures of pyrazolecarboxylic acid alkoxyamides with certain fungicidal or pesticidal compounds.

However the ecological and economic demands made on modem active ingredients, for example fungicides, are increasing constantly, for example with respect to activity spectrum, toxicity, selectivity, application rate, formation of residues and favourable manufacture.

Thus, there is a constant need for developing new, alternative plant protection products which in some areas at least help to fulfill the above-mentioned requirements. One way of fulfilling such need can be the development of novel compositions comprising of different fungicides which have advantages over the known compositions at least in some areas.

In view of this, it was in particular an object of the present invention to provide compositions which exhibit activity against harmful microorganisms in plants, in the protection of materials and show a positive effect on the plant physiology. Moreover, it was a further particular object of the present invention, to reduce the application rates and broaden the activity spectrum of the fungicides, and thereby to provide a composition which, preferably at a reduced total amount of active compounds applied, has improved activity against harmful microorganisms. In particular, it was a further object of the present invention to provide a composition which, when applied to a crop, results in a decreased amount of residues in the crop, and nevertheless provides efficient disease control.

It has now surprisingly been found that compositions comprising

(A) at least one compound of formula (I)

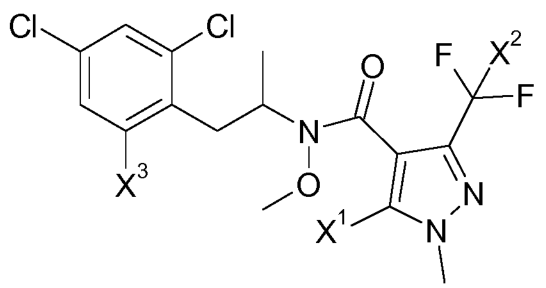

(I),

in which $X^1$, $X^2$ and $X^3$ independently from each other represent a hydrogen atom, a fluorine atom or a chlorine atom, or agrochemically acceptable salts or isomers or enantiomers or tautomers or N-oxides thereof, and

(B) at least one compound selected from the group consisting of

(B1) Quinoline derivatives selected from the group consisting of

Compound (B1-1)

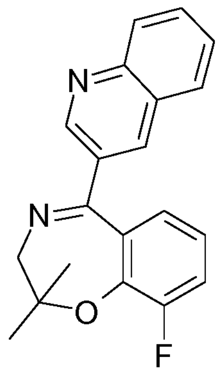

(B1-1)

(9-fluoro-2,2-dimethyl-5-(quinolin-3-yl)-2,3-dihydro-1,4-benzoxazepine),

Compound (B1-2)

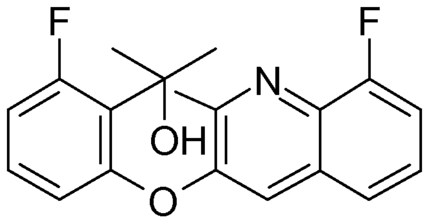

(B1-2)

(2-{2-fluoro-6-[(8-fluoro-2-methylquinolin-3-yl)oxy]phenyl}propan-2-ol),

Compound (B1-3)

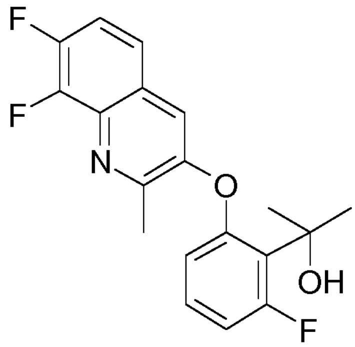

(B1-3)

(2-{2-[(7,8-difluoro-2-methylquinolin-3-yl)oxy]-6-fluorophenyl}propan-2-ol),

Compound (B1-4)

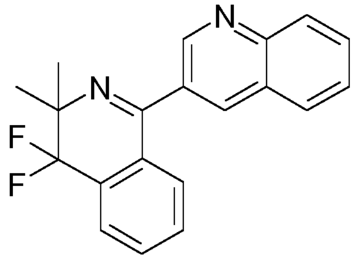

(B1-4)

(3-(4,4-difluoro-3,3-dimethyl-3,4-dihydroisoquinolin-1-yl)quinolone) are useful for controlling harmful microorganisms in plants.

In some embodiments, such compositions act in a synergistic fashion.

The invention also comprises a method for preparing an agricultural composition comprising adding agriculturally suitable components such as suitable extenders, solvents, spontaneity promoters, carriers, emulsifiers, dispersants, frost protectants, thickeners, adjuvants or the like to the composition according to the invention. Furthermore the invention comprises a method for reducing damage of plants and plant parts or losses in harvested fruits or vegetables caused by harmful microorganisms by controlling such harmful microorganisms, comprising applying the composition to the plant or the harmful microorganisms or the habitat of the plant or the habitat of the harmful microorganisms.

In view of this, the problem underlying the present invention has been solved by providing novel compositions which exhibit fungicidal and/ or synergistic activity against harmful microorganisms in plants, in the protection of materials and as plant growth regulators. Moreover, the novel compositions according to the invention enable reduced application rates and broaden the activity spectrum of the fungicides. Finally the novel compositions provide improved activity harmful microorganisms and consequently provide efficient disease control for reducing damage of plants and plant parts or losses in harvested fruits or vegetables.

Preference is given to compositions comprising those compounds of the formula (I), in which $X^2$ represents a hydrogen atom.

Particular preference is given to compositions comprising those compounds of the formula (I), in which $X^2$ represents a hydrogen atom and $X^3$ represents a chlorine atom.

Very particular preference is given to compositions comprising those compounds of the formula (I), in which $X^1$ represents a hydrogen atom, $X^2$ represents a hydrogen atom and $X^3$ represents a chlorine atom.

Preference is given to compositions comprising at least one compound of the formula (I) selected from the group consisting of:

Compound (I-1)

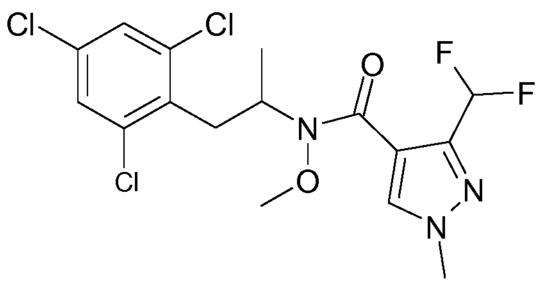

3-(difluoromethyl)-N-methoxy-1-methyl-N-[1-(2,4,6-trichlorophenyl)propan-2-yl]-1H-pyrazole-4-carboxamide,

Compound (I-2)

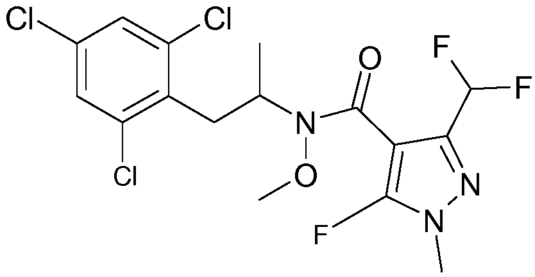

3-(difluoromethyl)-5-fluoro-N-methoxy-1-methyl-N-[1-(2,4,6-trichlorophenyl)propan-2-yl]-1H-pyrazole-4-carboxamide,

Compound (I-3)

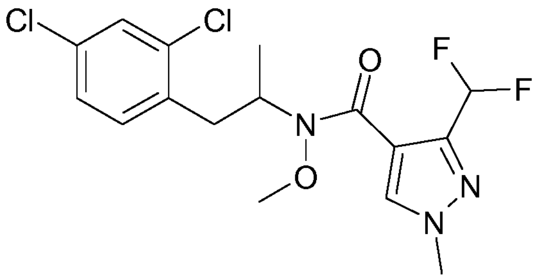

N-[1-(2,4-dichlorophenyl)propan-2-yl]-3-(difluoromethyl)-N-methoxy-1-methyl-1H-pyrazole-4-carboxamide,

Compound (I-4)

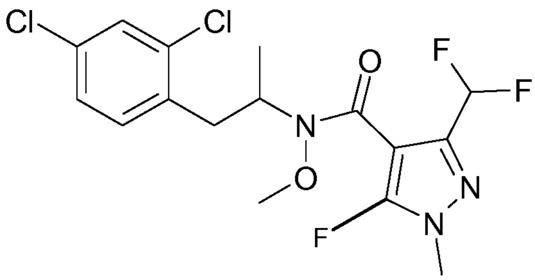

N-[1-(2,4-dichlorophenyl)propan-2-yl]-3-(difluoromethyl)-5-fluoro-N-methoxy-1-methyl-1H-pyrazole-4-carboxamide,

Compound (I-5)

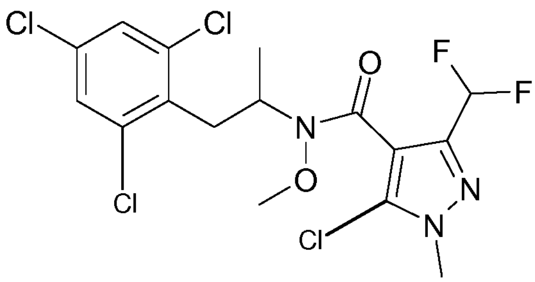

5-chloro-3-(difluoromethyl)-N-methoxy-1-methyl-N-[1-(2,4,6-trichlorophenyl)propan-2-yl]-1H-pyrazole-4-carboxamide,

Compound (I-6)

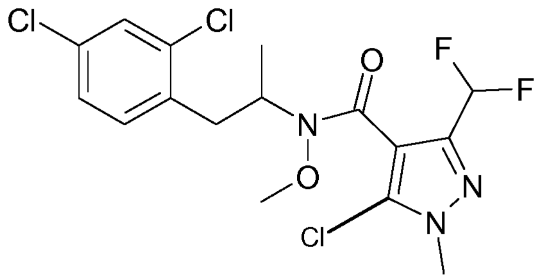

5-chloro-N-[1-(2,4-dichlorophenyl)propan-2-yl]-3-(difluoromethyl)-N-methoxy-1-methyl-1H-pyrazole-4-carboxamide.

Compound (I-2) is novel and be produced according to the process as outlined below.

The compound according to formula (I) comprises two enantiomeric forms; , The enantiomers can be present in racemic mixtures with a ratio of the (2R) and (2S) forms selected from the group consisting of 100:1 to 1:100 , 90:1 to 1: 90, 80:1 to 1: 80, 75:1 to 1: 75, 50:1 to 1: 50, 30:1 to 1: 30, 25:1 to 1: 25, 15:1 to 1: 15, 10:1 to 1: 10, 9:1 to 1: 9, 8:1 to 1: 8, 7:1 to 1: 7, 6:1 to 1: 6, 5:1 to 1: 5, 4:1 to 1: 4, 3:1 to 1:3, 2:1 to 1:2, and 1:1.

Preferred are ratios of 75:1 to 1: 75, 50:1 to 1:50, 30:1 to 1:30, 25:1 to 1: 25, 15:1 to 1: 15, 10:1 to 1:10, 9:1 to 1:9, 8:1 to 1: 8, 7:1 to 1: 7, 6:1 to 1: 6, 5:1 to 1: 5, 4:1 to 1: 4, 3:1 to 1:3,2:1 to 1:2, and 1:1.

More preferred are ratios 50:1 to 1:50, 30:1 to 1:30, 25:1 to 1: 25, 15:1 to 1: 15, 10:1 to 1: 10, 9:1 to 1: 9, 8:1 to 1: 8, 7:1 to 1: 7, 6:1 to 1: 6, 5:1 to 1: 5, 4:1 to 1: 4, 3:1 to 1:3,2:1 to 1:2, and 1:1.

Even more preference is given to compositions comprising at least one compound of the formula (I) selected from the group consisting of

Compound (I-1a)

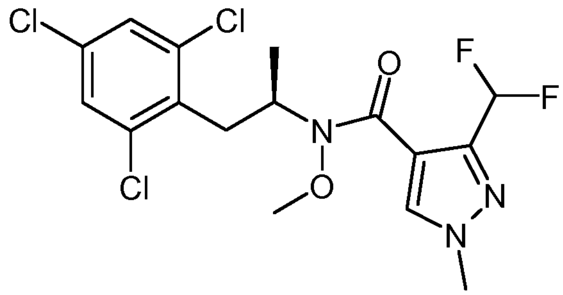

3-(difluoromethyl)-N-methoxy-1-methyl-N-[(2R)-1-(2,4,6-trichlorophenyl)propan-2-yl]-1H-pyrazole-4-carboxamide (I-1a),

Compound (I-1b)

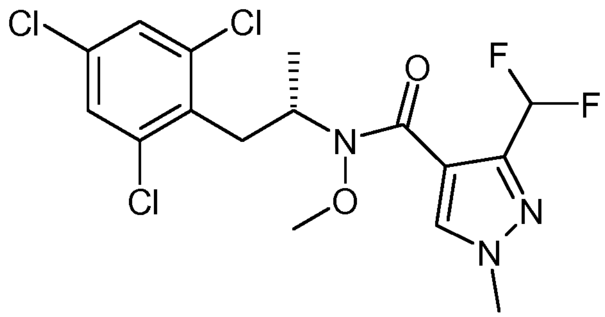

3-(difluoromethyl)-N-methoxy-1-methyl-N-[(2S)-1-(2,4,6-trichlorophenyl)propan-2-yl]-1H-pyrazole-4-carboxamide (I-1b),

Compound (I-2a)

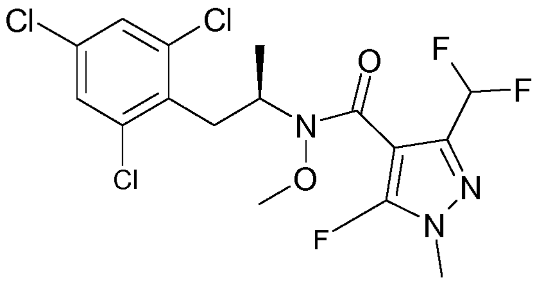

3-(difluoromethyl)-5-fluoro-N-methoxy-1-methyl-N-[(2R)-1-(2,4,6-trichlorophenyl)propan-2-yl]-1H-pyrazole-4-carboxamide (I-2a),

Compound (I-2b)

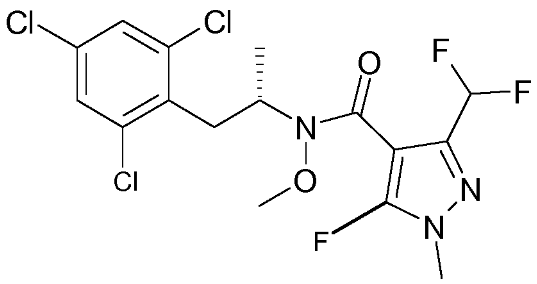

3-(difluoromethyl)-5-fluoro-N-methoxy-1-methyl-N-[(2S)-1-(2,4,6-trichlorophenyl)propan-2-yl]-1H-pyrazole-4-carboxamide (I-2b),

Compound (I-3a)

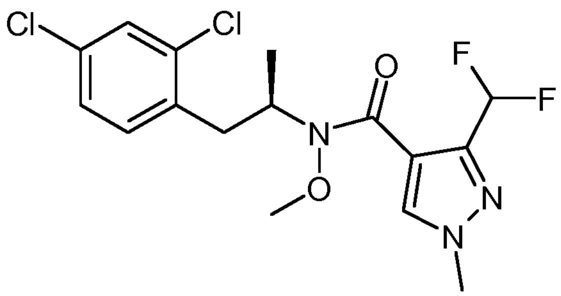

N-[(2R)-1-(2,4-dichlorophenyl)propan-2-yl]-3-(difluoromethyl)-N-methoxy-1-methyl-1H-pyrazole-4-carboxamide (I-3a),

Compound (I-3b)

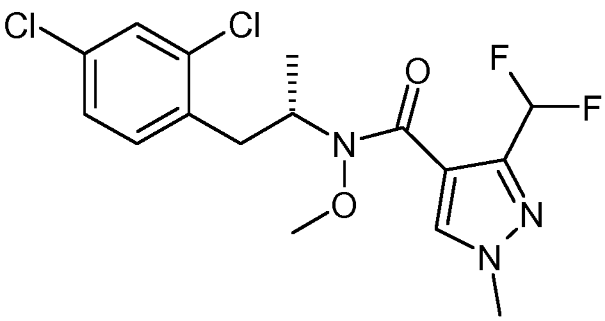

N-[(2S)-1-(2,4-dichlorophenyl)propan-2-yl]-3-(difluoromethyl)-N-methoxy-1-methyl-1H-pyrazole-4-carboxamide (I-3b),

Compound (I-4a)

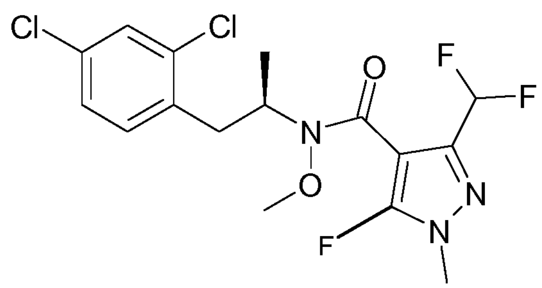

N-[(2R)-1-(2,4-dichlorophenyl)propan-2-yl]-3-(difluoromethyl)-5-fluoro-N-methoxy-1-methyl-1H-pyrazole-4-carboxamide (I-4a),

Compound (I-4b)

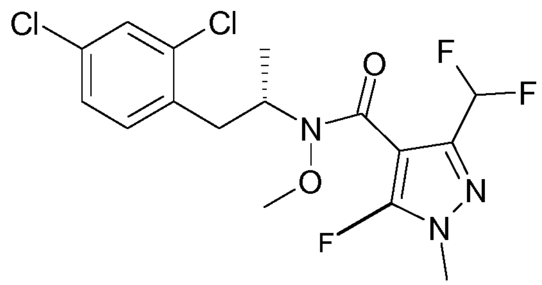

N-[(2S)-1-(2,4-dichlorophenyl)propan-2-yl]-3-(difluoromethyl)-5-fluoro-N-methoxy-1-methyl-1H-pyrazole-4-carboxamide (I-4b),

Compound (I-5a)

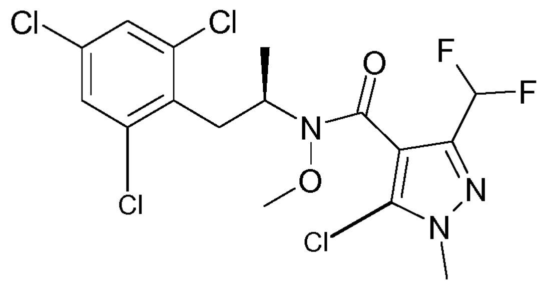

5-chloro-3-(difluoromethyl)-N-methoxy-1-methyl-N-[(2R)-1-(2,4,6-trichlorophenyl)propan-2-yl]-1H-pyrazole-4-carboxamide (I-5a),

Compound (I-5b)

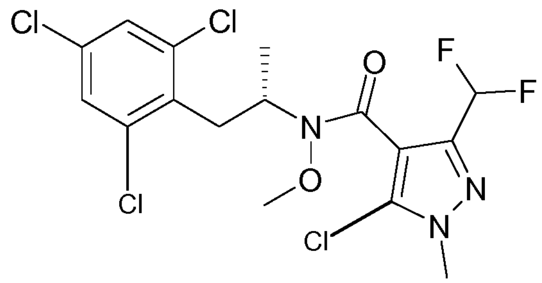

5-chloro-3-(difluoromethyl)-N-methoxy-1-methyl-N-[(2S)-1-(2,4,6-trichlorophenyl)propan-2-yl]-1H-pyrazole-4-carboxamide (I-5b),

Compound (I-6a)

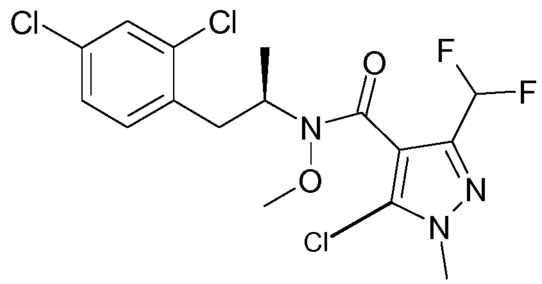

5-chloro-N-[(2R)-1-(2,4-dichlorophenyl)propan-2-yl]-3-(difluoromethyl)-N-methoxy-1-methyl-1H-pyrazole-4-carboxamide (I-6a),

Compound (I-6b)

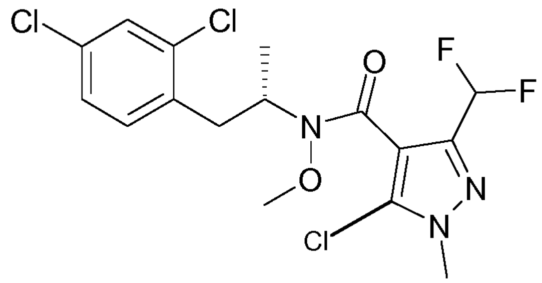

5-chloro-N-[(2S)-1-(2,4-dichlorophenyl)propan-2-yl]-3-(difluoromethyl)-N-methoxy-1-methyl-1H-pyrazole-4-carboxamide (I-6b).

Particularly preferred are the following compositions comprising the combinations of compounds according to formula (I) and component (B):

(I-1) + (B1-1), (I-1) + (B1-2), (I-1) + (B1-3), (I-1) + (B1-4), (I-2) + (B1-1), (I-2) + (B1-2), (I-2) + (B1-3), (I-2) + (B1-4), (I-3) + (B1-1), (I-3) + (B1-2), (I-3) + (B1-3), (I-3) + (B1-4), (I-4) + (B1-1), (I-4) + (B1-2), (I-4) + (B1-3), (I-4) + (B1-4), (I-5) + (B1-1), (I-5) + (B1-2), (I-5) + (B1-3), (I-5) + (B1-4), (I-6) + (B1-1), (I-6) + (B1-2), (I-6) + (B1-3), (I-6) + (B1-4);

(I-1a) + (B1-1), (I-1a) + (B1-2), (I-1a) + (B1-3), (I-1a) + (B1-4), (I-2a) + (B1-1), (I-2a) + (B1-2), (I-2a) + (B1-3), (I-2a) + (B1-4), (I-3a) + (B1-1), (I-3a) + (B1-2), (I-3a) + (B1-3), (I-3a) + (B1-4), (I-4a) + (B1-1), (I-4a) + (B1-2), (I-4a) + (B1-3), (I-4a) + (B1-4), (I-5a) + (B1-1), (I-5a) + (B1-2), (I-5a) + (B1-3), (I-5a) + (B1-4), (I-6a) + (B1-1), (I-6a) + (B1-2), (I-6a) + (B1-3), (I-6a) + (B1-4);

(I-1b) + (B1-1), (I-1b) + (B1-2), (I-1b) + (B1-3), (I-1b) + (B1-4), (I-2b) + (B1-1), (I-2b) + (B1-2), (I-2b) + (B1-3), (I-2b) + (B1-4), (I-3b) + (B1-1), (I-3b) + (B1-2), (I-3b) + (B1-3), (I-3b) + (B1-4), (I-4b) + (B1-1), (I-4b) + (B1-2), (I-4b) + (B1-3), (I-4b) + (B1-4), (I-5b) + (B1-1), (I-5b) + (B1-2), (I-5b) + (B1-3), (I-5b) + (B1-4), (I-6b) + (B1-1), (I-6b) + (B1-2), (I-6b) + (B1-3), (I-6b) + (B1-4).

In general, the weight ratio of component (A) to component (B) is from 2000 : 1 to 1 : 1000.
The weight ratio of compound (A) to compound (B) is preferably from 100 : 1 to 1 : 100; more preferably from 20 : 1 to 1 : 50. The active ingredient mixture of compound (A) to compound (B) comprises compounds of formula I and at least one compound (B) as described above preferably in a mixing ratio of from 1000:1 to 1:1000, very preferably from 50:1 to 1:50, more preferably in a ratio of from 20:1 to 1:20, even more preferably from 10:1 to 1:10, very preferably from 5:1 and 1:5, special preference being given to a ratio of from 2:1 to 1:2, and a ratio of from 4:1 25 to 2:1 being likewise preferred, above all in a ratio of 1:1, or 5:1, or 5:2, or 5:3, or 5:4, or 4:1, or 4:2, or 4:3, or 3:1, or 3:2, or 2:1, or 1 :5, or 2:5, or 3:5, or 4:5, or 1:4, or 2:4, or 3:4, or 1 :3, or 2:3, or 1 :2, or 1 :600, or 1 :300, or 1:150, or 1:35, or 2:35, or 4:35, or 1 :75, or 2:75, or 4:75, or 1:6000, or 1:3000, or 1:1500, or 1:350, or 2:350, or 4:350, or 1:750, or 2:750, or 4:750. Those

mixing ratios are understood to include, on the one hand, ratios by weight and also, on other hand, molar ratios.
It has been found, surprisingly, that certain weight ratios of compound (A) to compound (B) are able to give rise to synergistic activity. Therefore, a further aspect of the invention are compositions, wherein compound (A) and compound (B) are present in the composition in amounts producing a synergistic effect. This synergistic activity is apparent from the fact that the activity of the composition for controlling harmful microorganisms comprising compound (A) and compound (B) is greater than the sum of the these activities of compound (A) and of compound (B).
This synergistic activity extends the range of action of compound (A) and compound (B) in two ways. Firstly, the rates of application of compound (A) and compound (B) are lowered whilst the action remains equally good, meaning that the active ingredient mixture still achieves a high degree of control of harmful microorganisms even where the two individual components have become totally ineffective in such a low application rate range. Secondly, there is a substantial broadening of the spectrum of harmful microorganisms that can be controlled.

Definitions

In the context of the present invention "harmful microorganisms" are phytopathogenic fungi, phytopathogenic bacteria, phytopathogenic oomycetes, and phytopathogenic viruses. "Phytopathogenic" means that the respective organism is capable of infesting plants or plant parts. Phytopathogenic" means also that the respective organism is capable of infesting a seed of a plant, a plant propagation material or a plant product.

Preferably phytopathogenic microorganism means a phytopathogenic fungus.

Fungi means Plasmodiophoromycetes, Peronosporomycetes (Syn. Oomycetes), Chytridiomycetes, Zygomycetes, Ascomycetes, Basidiomycetes and Deuteromycetes (Syn. Fungi imperfecti).

Bacteria means bacterial species including Pseudomonadaceae, Rhiwbiaceae, Enterobacteriaceae, Corynebacteriaceae and Streptomycetaceae capable of infecting a plant, a seed of a plant, a plant propagation material or a plant product.

Throughout this document the expression "composition" stands for the various mixtures or combinations of compound (A) and (B), for example in a single "ready-mix" form, in a combined spray mixture composed from separate formulations of the single active ingredient components, such as a "tank-mix", and in a combined use of the single active ingredients when applied in a sequential manner, i.e. one after the other with a reasonably short period, such as a few hours or days. The order of applying the compound (A) and (B) is not essential for working the present invention. Throughout this document the expression "composition" stands for the various mixtures or combinations of compound (A), a compound according to formula (I), and (B), for example in a single "ready-mix" form, in a combined spray mixture composed from separate formulations of the single active ingredient components, such as a "tank-mix", and in a combined use of the single active ingredients when applied in a sequential manner, i.e. one after the other with a reasonably short period, such as a few hours or days. The order of applying the compound (A), a compound according to formula (I), and (B) is not essential for working the present invention. Another example of a combination of compound (A), a compound according to formula (I), and (B) according to the invention is that compound (A), a compound according to formula (I), and (B) are not present together in the same formulation, but packaged separately (combipack), i.e., not jointly preformulated. As such, combipacks include one or more separate containers such as vials, cans, bottles, pouches, bags or canisters, each container containing a separate component for an agrochemical composition, here compound (A), a compound according to formula (I), and (B). One example is a two-component combipack. Accordingly the present invention also relates to a two-component combipack, comprising a first component which in turn comprises a compound of formula (I)/mixing partner (A), a liquid or solid carrier and, if appropriate, at least one surfactant and/or at least one customary auxiliary, and a second component which in turn comprises mixing partner (B), a liquid or solid carrier and, if appropriate, at least one surfactant and/or at least one customary auxiliary. More details, e.g. as to suitable liquid and solid carriers, surfactants and customary auxiliaries are described below.

In the context of the present invention, "control of harmful microorganisms" means a reduction in infestation by harmful microorganisms, compared with the untreated plant or plant part as defined below measured as fungicidal efficacy, preferably a reduction by 25-50 %, compared with the untreated plant (100 %), more preferably a reduction by 40-79 %, compared with the untreated plant (100 %); even more preferably, the infection by harmful microorganisms is entirely suppressed (by 70-100 %). The control may be curative, i.e. for treatment of already infected plants, or protective, for protection of plants which have not yet been infected.

An "effective but non-phytotoxic amount" means an amount of the inventive composition which is sufficient to control the fungal disease of the plant in a satisfactory manner or to eradicate the fungal disease completely, and which, at the same time, does not cause any significant symptoms of phytotoxicity. In general, this application rate may vary within a relatively wide range. It depends on several factors, for example on the fungus to be controlled, the plant, the climatic conditions and the ingredients of the inventive compositions.

Suitable organic solvents include all polar and non-polar organic solvents usually employed for formulation purposes. Preferable the solvents are selected from ketones, e.g. methyl-isobutyl-ketone and cyclohexanone, amides,

e.g. dimethyl formamide and alkanecarboxylic acid amides, e.g. N,N-dimethyl decaneamide and N,N-dimethyl octanamide, furthermore cyclic solvents, e.g. N-methyl-pyrrolidone, N-octyl-pyrrolidone, N-dodecyl-pyrrolidone, N-octyl-caprolactame, N-dodecyl-caprolactame and butyrolactone, furthermore strong polar solvents, e.g. dimethylsulfoxide, and aromatic hydrocarbons, e.g. xylol, Solvesso™, mineral oils, e.g. white spirit, petroleum, alkyl benzenes and spindle oil, also esters, e.g. propyleneglycol-monomethylether acetate, adipic acid dibutylester, acetic acid hexylester, acetic acid heptylester, citric acid tri-*n*-butylester and phthalic acid di-*n*-butylester, and also alkohols, e.g. benzyl alcohol and 1-methoxy-2-propanol.

According to the invention, a carrier is a natural or synthetic, organic or inorganic substance with which the active ingredients are mixed or combined for better applicability, in particular for application to plants or plant parts or seed. The carrier, which may be solid or liquid, is generally inert and should be suitable for use in agriculture.

Useful solid or liquid carriers include: for example ammonium salts and natural rock dusts, such as kaolins, clays, talc, chalk, quartz, attapulgite, montmorillonite or diatomaceous earth, and synthetic rock dusts, such as finely divided silica, alumina and natural or synthetic silicates, resins, waxes, solid fertilizers, water, alcohols, especially butanol, organic solvents, mineral and vegetable oils, and derivatives thereof. Mixtures of such carriers can likewise be used.

Suitable solid filler and carrier include inorganic particles, e.g. carbonates, silikates, sulphates and oxides with an average particle size of between 0.005 and 20 $\mu$m, preferably of between 0.02 to 10 $\mu$m, for example ammonium sulphate, ammonium phosphate, urea, calcium carbonate, calcium sulphate, magnesium sulphate, magnesium oxide, aluminium oxide, silicium dioxide, so-called fine-particle silica, silica gels, natural or synthetic silicates, and alumosilicates and plant products like cereal flour, wood powder/sawdust and cellulose powder.

Useful solid carriers for granules include: for example crushed and fractionated natural rocks such as calcite, marble, pumice, sepiolite, dolomite, and synthetic granules of inorganic and organic meals, and also granules of organic material such as sawdust, coconut shells, maize cobs and tobacco stalks.

Useful liquefied gaseous extenders or carriers are those liquids which are gaseous at standard temperature and under standard pressure, for example aerosol propellants such as halohydrocarbons, and also butane, propane, nitrogen and carbon dioxide.

In the formulations, it is possible to use tackifiers such as carboxymethylcellulose, and natural and synthetic polymers in the form of powders, granules or latices, such as gum arabic, polyvinyl alcohol and polyvinyl acetate, or else natural phospholipids, such as cephalins and lecithins, and synthetic phospholipids. Further additives may be mineral and vegetable oils.

If the extender used is water, it is also possible to employ, for example, organic solvents as auxiliary solvents. Useful liquid solvents are essentially: aromatics such as xylene, toluene or alkylnaphthalenes, chlorinated aromatics and chlorinated aliphatic hydrocarbons such as chlorobenzenes, chloroethylenes or dichloromethane, aliphatic hydrocarbons such as cyclohexane or paraffins, for example mineral oil fractions, mineral and vegetable oils, alcohols such as butanol or glycol and their ethers and esters, ketones such as acetone, methyl ethyl ketone, methyl isobutyl ketone or cyclohexanone, strongly polar solvents such as dimethylformamide and dimethyl sulphoxide, and also water.

The inventive compositions may additionally comprise further components, for example surfactants. Useful surfactants are emulsifiers and/or foam formers, dispersants or wetting agents having ionic or nonionic properties, or mixtures of these surfactants. Examples of these are salts of polyacrylic acid, salts of lignosulphonic acid, salts of phenolsulphonic acid or naphthalenesulphonic acid, polycondensates of ethylene oxide with fatty alcohols or with fatty acids or with fatty amines, substituted phenols (preferably alkylphenols or arylphenols), salts of sulphosuccinic esters, taurine derivatives (preferably alkyl taurates), phosphoric esters of polyethoxylated alcohols or phenols, fatty esters of polyols, and derivatives of the compounds containing sulphates, sulphonates and phosphates, for example alkylaryl polyglycol ethers, alkylsulphonates, alkylsulphates, arylsulphonates, protein hydrolysates, lignosulphite waste liquors and methylcellulose. The presence of a surfactant is necessary if one of the active ingredients and/or one of the inert carriers is insoluble in water and when application is effected in water. The proportion of surfactants is between 5 and 40 per cent by weight of the inventive composition.

Suitable surfactants (adjuvants, emulsifiers, dispersants, protective colloids, wetting agent and adhesive) include all common ionic and non-ionic substances, for example ethoxylated nonylphenols, polyalkylene glycolether of linear or branched alcohols, reaction products of alkyl phenols with ethylene oxide and/or propylene oxide, reaction products of fatty acid amines with ethylene oxide and/or propylene oxide, furthermore fattic acid esters, alkyl sulfonates, alkyl sulphates, alkyl ethersulphates, alkyl etherphosphates, arylsulphate, ethoxylated arylalkylphenols, e.g. tristyrylphenol-ethoxylates, furthermore ethoxylated and propoxylated arylalkylphenols like sulphated or phosphated arylalkylphenol-ethoxylates and -ethoxy- and -propoxylates. Further examples are natural and synthetic, water soluble polymers, e.g. lignosulphonates, gelatine, gum arabic, phospholipides, starch, hydrophobic modified starch and cellulose derivatives, in particular cellulose ester and cellulose ether, further polyvinyl alcohol, polyvinyl acetate, polyvinyl pyrrolidone, polyacrylic acid, polymethacrylic acid and co-polymerisates of (meth)acrylic acid and (meth)acrylic acid esters, and further co-polymerisates of methacrylic acid and methacrylic acid esters which are neutralized with alkalimetal hydroxide and also condensation products of optionally substituted naphthalene sulfonic acid salts with formaldehyde.

It is possible to use dyes such as inorganic pigments, for example iron oxide, titanium oxide and Prussian Blue, and organic dyes such as alizarin dyes, azo dyes and metal phthalocyanine dyes, and trace nutrients such as salts of iron, manganese, boron, copper, cobalt, molybdenum and zinc.

Antifoams which may be present in the formulations include e.g. silicone emulsions, longchain alcohols, fattiy acids and their salts as well as fluoroorganic substances and mixtures therof.

Examples of thickeners are polysaccharides, e.g. xanthan gum or veegum, silicates, e.g. attapulgite, bentonite as well as fine-particle silica.

If appropriate, it is also possible for other additional components to be present, for example protective colloids, binders, adhesives, thickeners, thixotropic substances, penetrants, stabilizers, sequestrants, complexing agents. In general, the active ingredients can be combined with any solid or liquid additive commonly used for formulation purposes.

The inventive compositions can be used as such or, depending on their particular physical and/or chemical properties, in the form of their formulations or the use forms prepared therefrom, such as aerosols, capsule suspensions, cold-fogging concentrates, warm-fogging concentrates, encapsulated granules, fine granules, flowable concentrates for the treatment of seed, ready-to-use solutions, dustable powders, emulsifiable concentrates, oil-in-water emulsions, water-in-oil emulsions, macrogranules, microgranules, oil-dispersible powders, oil-miscible flowable concentrates, oil-miscible liquids, gas (under pressure), gas generating product, foams, pastes, pesticide coated seed, suspension concentrates, suspoemulsion concentrates, soluble concentrates, suspensions, wettable powders, soluble powders, dusts and granules, water-soluble and water-dispersible granules or tablets, water-soluble and water-dispersible powders for the treatment of seed, wettable powders, natural products and synthetic substances impregnated with active ingredient, and also microencapsulations in polymeric substances and in coating materials for seed, and also ULV cold-fogging and warm-fogging formulations.

The inventive compositions include not only formulations which are already ready for use and can be applied with a suitable apparatus to the plant or the seed, but also commercial concentrates which have to be diluted with water prior to use. Customary applications are for example dilution in water and subsequent spraying of the resulting spray liquor, application after dilution in oil, direct application without dilution, seed treatment or soil application of granules.

The inventive compositions and formulations generally contain between 0.05 and 99 % by weight, preferably 0.01 and 98 % by weight, more preferably between 0.1 and 95 % by weight, even more preferably between 0.5 and 90 % of active ingredient, most preferably between 10 and 70 % by weight. For special applications, e.g. for protection of wood and derived timber products the inventive compositions and formulations generally contain between 0.0001 and 95 % by weight, preferably 0.001 to 60 % by weight of active ingredient.

The formulations mentioned can be prepared in a manner known per se, for example by mixing the active ingredients with at least one customary extender, solvent or diluent, adjuvant, emulsifier, dispersant, and/or binder or fixative, wetting agent, water repellent, if appropriate desiccants and UV stabilizers and, if appropriate, dyes and pigments, antifoams, preservatives, inorganic and organic thickeners, adhesives, gibberellins and also further processing auxiliaries and also water. Depending on the formulation type to be prepared further processing steps are necessary, e.g. wet grinding, dry grinding and granulation.

The compositions according to the invention may be present as such or in their (commercial) formulations and in the use forms prepared from these formulations as a mixture with other (known) active ingredients, such as insecticides, attractants, sterilants, bactericides, acaricides, nematicides, fungicides, growth regulators, herbicides, fertilizers, safeners and/or semiochemicals.

The inventive treatment of the plants and plant parts with the active ingredients or compositions is effected directly or by action on their surroundings, habitat or storage space by the customary treatment methods, for example by dipping, spraying, atomizing, irrigating, evaporating, dusting, fogging, broadcasting, foaming, painting, spreadingon, watering (drenching), drip irrigating and, in the case of propagation material, especially in the case of seeds, also by dry seed treatment, wet seed treatment, slurry treatment, incrustation, coating with one or more coats, etc. It is also possible to deploy the active ingredients by the ultra-low volume method or to inject the active ingredient preparation or the active ingredient itself into the soil.

*Plant/Crop Protection*

The compositions have potent microbicidal activity and can be used for control of harmful microorganisms, such as fungi and bacteria, in crop protection and in the protection of materials.

The invention also relates to a method for controlling harmful microorganisms, characterized in that the compositions according to the invention are applied to the harmful microorganims and/or their habitat.

Fungicides can be used in crop protection for control of phytopathogenic fungi. They are characterized by an outstanding efficacy against a broad spectrum of phytopathogenic fungi, including soilborne pathogens, which are in particular members of the classes *Plasmodiophoromycetes*, *Peronosporomycetes* (Syn. *Oomycetes*), *Chytridiomycetes, Zygomycetes, Ascomycetes, Basidiomycetes* and *Deuteromycetes* (Syn. *Fungi imperfecti*). Some fungicides are systemically active and ca be used in plant protection as foliar, seed dressing or soil fungicide. Furthermore, they are suitable for combating fungi, which inter alia infest wood or roots of plant.

Bactericides can be used in crop protection for control of phytopathogenic bacteria. They are characterized by an outstanding efficacy against a broad spectrum of phytopathogenic bacteria, including *Pseudomonadaceae, Rhizobiaceae, Enterobacteriaceae, Corynebacteriaceae* and *Streptomycetaceae.*

Non-limiting examples of pathogens of fungal diseases which can be treated in accordance with the invention include:

diseases caused by powdery mildew pathogens, for example *Blumeria* species, for example *Blumeria* graminis; *Podosphaera* species, for example *Podosphaera leucotricha; Sphaerotheca* species, for example *Sphaerotheca fuliginea*; *Uncinula* species, for example *Uncinula necator*;

diseases caused by rust disease pathogens, for example *Gymnosporangium* species, for example *Gymnosporangium sabinae*; *Hemileia* species, for example *Hemileia vastatrix*; *Phakopsora* species, for example *Phakopsora pachyrhizi* and *Phakopsora meibomiae*; *Puccinia* species, for example *Puccinia recondite*, *P. triticina, P. graminis* or *P. striiformis*; *Uromyces* species, for example *Uromyces appendiculatus*;

diseases caused by pathogens from the group of the *Oomycetes*, for example *Albugo* species, for example *Algubo candida*; *Bremia* species, for example *Bremia lactucae*; *Peronospora* species, for example *Peronospora pisi* or *P. brassicae*; Phytophthora species, for example *Phytophthora infestans*; *Plasmopara* species, for example *Plasmopara viticola*; *Pseudoperonospora* species, for example *Pseudoperonospora humuli* or *Pseudoperonospora cubensis*; Pythium species, for example *Pythium ultimum*;

leaf blotch diseases and leaf wilt diseases caused, for example, by *Alternaria* species, for example *Alternaria solani*; *Cercospora* species, for example *Cercospora beticola*; *Cladiosporium* species, for example *Cladiosporium cucumerinum; Cochliobolus* species, for example *Cochliobolus sativus* (conidia form: Drechslera, Syn: Helminthosporium), *Cochliobolus miyabeanus*; *Colletotrichum* species, for example *Colletotrichum lindemuthanium; Cycloconium* species, for example *Cycloconium oleaginum*; *Diaporthe* species, for example *Diaporthe citri*; *Elsinoe* species, for example *Elsinoe fawcettii*; *Gloeosporium* species, for example *Gloeosporium laeticolor*; *Glomerella* species, for example *Glomerella cingulata*; *Guignardia* species, for example *Guignardia bidwelli*; *Leptosphaeria* species, for example *Leptosphaeria maculans, Leptosphaeria nodorum*; *Magnaporthe* species, for example *Magnaporthe grisea*; *Microdochium* species, for example *Microdochium nivale*; *Mycosphaerella* species, for example *Mycosphaerella graminicola, M. arachidicola* and *M. fijiensis*; *Phaeosphaeria* species, for example *Phaeosphaeria nodorum*; *Pyrenophora* species, for example *Pyrenophora teres, Pyrenophora tritici repentis*; *Ramularia* species, for example *Ramularia collo-cygni, Ramularia areola*; *Rhynchosporium* species, for example *Rhynchosporium secalis*; *Septoria* species, for example *Septoria apii*, *Septoria lycopersii*; *Typhula* species, for example *Typhula incarnata*; *Venturia* species, for example *Venturia inaequalis*;

root and stem diseases caused, for example, by *Corticium* species, for example *Corticium graminearum*; *Fusarium* species, for example *Fusarium oxysporum*; *Gaeumannomyces* species, for example *Gaeumannomyces graminis*; *Rhizoctonia* species, such as, for example *Rhizoctonia solani*; *Sarocladium* diseases caused for example by *Sarocladium oryzae*; *Sclerotium* diseases caused for example by *Sclerotium oryzae*; *Tapesia* species, for example *Tapesia acuformis*; *Thielaviopsis* species, for example *Thielaviopsis basicola*;

ear and panicle diseases (including corn cobs) caused, for example, by *Alternaria* species, for example *Alternaria* spp.; *Aspergillus* species, for example *Aspergillus flavus*; *Cladosporium* species, for example *Cladosporium cladosporioides*; *Claviceps* species, for example *Claviceps purpurea*; *Fusarium* species, for example *Fusarium culmorum*; *Gibberella* species, for example *Gibberella zeae*; *Monographella* species, for example *Monographella nivalis*; *Septoria* species, for example *Septoria nodorum*;

diseases caused by smut fungi, for example *Sphacelotheca* species, for example *Sphacelotheca reiliana*; *Tilletia* species, for example *Tilletia caries*, *T. controversa*; *Urocystis* species, for example *Urocystis occulta*; *Ustilago* species, for example *Ustilago nuda*, *U. nuda tritici*;

fruit rot caused, for example, by *Aspergillus* species, for example *Aspergillus flavus*; *Botrytis* species, for example *Botrytis cinerea*; *Penicillium* species, for example *Penicillium expansum* and *P. purpurogenum*; *Sclerotinia* species, for example *Sclerotinia sclerotiorum*; *Verticilium* species, for example *Verticilium alboatrum*;

seed and soilborne decay, mould, wilt, rot and damping-off diseases caused, for example, by *Alternaria* species, caused for example by *Alternaria brassicicola*; *Aphanomyces* species, caused for example by *Aphanomyces euteiches*; *Ascochyta* species, caused for example by *Ascochyta lentis*; *Aspergillus* species, caused for example by *Aspergillus flavus*; *Cladosporium* species, caused for example by *Cladosporium herbarum*; *Cochliobolus* species, caused for example by *Cochliobolus sativus*; (Conidiaform: Drechslera, Bipolaris Syn: Helminthosporium); *Colletotrichum* species, caused for example by *Colletotrichum coccodes*; *Fusarium* species, caused for example by *Fusarium culmorum*; *Gibberella* species, caused for example by *Gibberella zeae*; *Macrophomina* species, caused for example by *Macrophomina phaseolina*; *Monographella* species, caused for example by *Monographella nivalis*;

*Penicillium* species, caused for example by *Penicillium expansum*; *Phoma* species, caused for example by *Phoma lingam; Phomopsis* species, caused for example by *Phomopsis sojae; Phytophthora* species, caused for example by *Phytophthora cactorum; Pyrenophora* species, caused for example by *Pyrenophora graminea; Pyricularia* species, caused for example by *Pyricularia oryzae; Pythium* species, caused for example by *Pythium ultimum; Rhizoctonia* species, caused for example by *Rhizoctonia solani; Rhizopus* species, caused for example by *Rhizopus oryzae; Sclerotium* species, caused for example by *Sclerotium rolfsii; Septoria* species, caused for example by *Septoria nodorum; Typhula* species, caused for example by *Typhula incarnata; Verticillium* species, caused for example by *Verticillium dahliae;*

cancers, galls and witches' broom caused, for example, by *Nectria* species, for example *Nectria galligena;* wilt diseases caused, for example, by *Monilinia* species, for example *Monilinia laxa;*

leaf blister or leaf curl diseases caused, for example, by *Exobasidium* species, for example *Exobasidium vexans; Taphrina* species, for example *Taphrina deformans;*

decline diseases of wooden plants caused, for example, by Esca disease, caused for example by *Phaemoniella clamydospora, Phaeoacremonium aleophilum* and *Fomitiporia mediterranea;* Eutypa dyeback, caused for example by *Eutypa lata;* Ganoderma diseases caused for example by *Ganoderma boninense;* Rigidoporus diseases caused for example by *Rigidoporus lignosus;*

diseases of flowers and seeds caused, for example, by *Botrytis* species, for example *Botrytis cinerea;*

diseases of plant tubers caused, for example, by *Rhizoctonia* species, for example *Rhizoctonia solani; Helminthosporium* species, for example *Helminthosporium solani;*

Club root caused, for example, by *Plasmodiophora* species, for example *Plamodiophora brassicae;*

diseases caused by bacterial pathogens, for example *Xanthomonas* species, for example *Xanthomonas campestris pv. oryzae; Pseudomonas* species, for example *Pseudomonas syringae pv. lachrymans; Erwinia* species, for example *Erwinia amylovora.*

The following diseases of soya beans can be controlled with preference:

Fungal diseases on leaves, stems, pods and seeds caused, for example, by *Alternaria* leaf spot (*Alternaria spec. atrans tenuissima*), Anthracnose (*Colletotrichum gloeosporoides dematium var. truncatum*), brown spot (*Septoria glycines*), cercospora leaf spot and blight (*Cercospora kikuchii*), choanephora leaf blight (*Choanephora infundibulifera trispora* (Syn.)), dactuliophora leaf spot (*Dactuliophora glycines*), downy mildew (*Peronospora manshurica*), drechslera blight (*Drechslera glycini*), frogeye leaf spot (*Cercospora sojina*), leptosphaerulina leaf spot (*Leptosphaerulina trifolii*), phyllostica leaf spot (*Phyllosticta sojaecola*), pod and stem blight (*Phomopsis sojae*), powdery mildew (*Microsphaera diffusa*), pyrenochaeta leaf spot (*Pyrenochaeta glycines*), rhizoctonia aerial, foliage, and web blight (*Rhizoctonia solani*), rust (*Phakopsora pachyrhizi, Phakopsora meibomiae*), scab (*Sphaceloma glycines*), stemphylium leaf blight (*Stemphylium botryosum*), target spot (*Corynespora cassiicola*).

Fungal diseases on roots and the stem base caused, for example, by black root rot (*Calonectria crotalariae),* charcoal rot (*Macrophomina phaseolina*), fusarium blight or wilt, root rot, and pod and collar rot (*Fusarium oxysporum, Fusarium orthoceras, Fusarium semitectum, Fusarium equiseti*), mycoleptodiscus root rot (*Mycoleptodiscus terrestris*), neocosmospora (*Neocosmospora vasinfecta*), pod and stem blight (*Diaporthe phaseolorum*), stem canker (*Diaporthe phaseolorum var. caulivora*), phytophthora rot (*Phytophthora megasperma*), brown stem rot (*Phialophora gregata*), pythium rot (*Pythium aphanidermatum, Pythium irregulare, Pythium debaryanum, Pythium myriotylum, Pythium ultimum*), rhizoctonia root rot, stem decay, and damping-off (*Rhizoctonia solani*), sclerotinia stem decay (*Sclerotinia sclerotiorum*), sclerotinia southern blight (*Sclerotinia rolfsii*), thielaviopsis root rot (*Thielaviopsis basicola*).

The compositions according to the inventions can be used for curative or protective/preventive control of harmful microorganisms. The invention therefore also relates to curative and protective methods for controlling harmful microorganisms by the use of the compositions according to the invention , which are applied to the seed, the plant or plant parts, the fruit or the soil in which the plants grow.

The fact that the active ingredients are well tolerated by plants at the concentrations required for controlling harmful microorganisms allows the treatment of above-ground parts of plants, of propagation stock and seeds, and of the soil.

According to the invention all plants and plant parts can be treated. By plants is meant all plants and plant populations such as desirable and undesirable wild plants, cultivars and plant varieties (whether or not protectable by plant variety or plant breeder's rights). Cultivars and plant varieties can be plants obtained by conventional propagation and breeding methods which can be assisted or supplemented by one or more biotechnological methods such as by use of double haploids, protoplast fusion, random and directed mutagenesis, molecular or genetic markers or by bioengineering and genetic engineering methods. By plant parts is meant all above ground and below ground parts and organs of plants such as shoot, leaf, blossom and root, whereby for example leaves, needles, stems, branches, blossoms,

fruiting bodies, fruits and seed as well as roots, corms and rhizomes are listed. Crops and vegetative and generative propagating material, for example cuttings, corms, rhizomes, runners, slips and seeds also belong to plant parts.

The compositions according to the invention , when they are well tolerated by plants, have favourable homeotherm toxicity and are well tolerated by the environment, are suitable for protecting plants and plant parts, for enhancing harvest yields, for improving the quality of the harvested material. They can preferably be used as crop protection compositions. They are active against normally sensitive and resistant species and against all or some stages of development.

Plants which can be treated in accordance with the invention include the following main crop plants: maize, soya bean, alfalfa, cotton, sunflower, *Brassica* oil seeds such as *Brassica napus* (e.g. canola, rapeseed), *Brassica rapa, B. juncea* (e.g. (field) mustard) and *Brassica carinata, Arecaceae sp.* (e.g. oilpalm, coconut), rice, wheat, sugar beet, sugar cane, oats, rye, barley, millet and sorghum, triticale, flax, nuts, grapes and vine and various fruit and vegetables from various botanic taxa, e.g. *Rosaceae sp.* (e.g. pome fruits such as apples and pears, but also stone fruits such as apricots, cherries, almonds, plums and peaches, and berry fruits such as strawberries, raspberries, red and black currant and gooseberry), *Ribesioidae sp., Juglandaceae sp., Betulaceae sp., Anacardiaceae sp., Fagaceae sp., Moraceae sp., Oleaceae sp.* (e.g. olive tree), *Actinidaceae sp., Lauraceae sp.* (e.g. avocado, cinnamon, camphor), *Musaceae sp.* (e.g. banana trees and plantations), *Rubiaceae sp.* (e.g. coffee), *Theaceae sp.* (e.g. tea), *Sterculiceae sp., Rutaceae sp.* (e.g. lemons, oranges, mandarins and grapefruit); *Solanaceae sp.* (e.g. tomatoes, potatoes, peppers, capsicum, aubergines, tobacco), *Liliaceae sp., Compositae sp.* (e.g. lettuce, artichokes and chicory - including root chicory, endive or common chicory), *Umbelliferae sp.* (e.g. carrots, parsley, celery and celeriac), *Cucurbitaceae sp.* (e.g. cucumbers - including gherkins, pumpkins, watermelons, calabashes and melons), *Alliaceae sp.* (e.g. leeks and onions), *Cruciferae sp.* (e.g. white cabbage, red cabbage, broccoli, cauliflower, Brussels sprouts, pak choi, kohlrabi, radishes, horseradish, cress and chinese cabbage), *Leguminosae sp.* (e.g. peanuts, peas, lentils and beans - e.g. common beans and broad beans), *Chenopodiaceae sp.* (e.g. Swiss chard, fodder beet, spinach, beetroot), *Linaceae sp.* (e.g. hemp), *Cannabeacea sp.* (e.g. cannabis), *Malvaceae sp.* (e.g. okra, cocoa), *Papaveraceae* (e.g. poppy), *Asparagaceae* (e.g. asparagus); useful plants and ornamental plants in the garden and woods including turf, lawn, grass and *Stevia rebaudiana;* and in each case genetically modified types of these plants.

Alfafa;
beets, for example sugar beet and fodder beet;
cereals, for example barley, maize/corn, millet/sorghum, oats, rice, rye, triticale, wheat;
cucurbits, for example pumpkin/squash, gherkins, calabashes, cucumbers and melons;
fibre plants, for example cotton, flax, hemp, cannabis and jute;
fruits, for example
pome fruit, for example apples, pears, quince;
berries, for example Ribesioidae sp. Like strawberries, raspberries, blackberries, blueberries, red and black currant and gooseberry;
citrus fruit, for example oranges, lemons, grapefruit and tangerines;
stone fruit, for example peaches, mangos, nectarines, cherries, plums, common plums, apricots;
legumes, for example beans, lentils, peas and soybeans;
oil crops, for example Brassica napus (canola, rapeseed, oilseed rape), Brassica rapa, B. juncea (e.g. (field) mustard), Brassica carinata, Arecaceae sp. (e.g. oilpalm, coconut), poppies, olives, sunflowers, coconuts, castor oil plants, cacao and peanuts, Oleaceae sp. (e.g. olive tree, olives);
Malvaceae sp. (e.g. okra, cocoa);
Manihoteae sp. (for instance Manihot esculenta, manioc),
Musaceae sp. (e.g. banana trees, bananas and plantations),
nuts of various botanical taxa such as peanuts, Juglandaceae sp.(Walnut, Persian Walnut (Juglans regia), Buttemut (Juglans), Hickory, Shagbark Hickory, Pecan (Carya), Wingnut (Pterocarya)), Fagaceae sp. (Chestnut (Castanea), Chestnuts, including Chinese Chestnut, Malabar chestnut, Sweet Chestnut, Beech (Fagus), Oak (Quercus), Stoneoak, Tanoak (Lithocarpus)); Betulaceae sp. (Alder (Alnus), Birch (Betula), Hazel, Filbert (Corylus), Hornbeam), Leguminosae sp. (for instance peanuts, peas and beans beans - such as climbing beans and broad beans), Asteraceae sp. (for instance sunflower seed), Almond, Beech, Butternut, Brazil nut, Candlenut, Cashew, Colocynth, Cotton seed, Cucurbita ficifolia, Filbert, Indian Beech or Pongam Tree, Kola nut, Lotus seed, Macadamia, Mamoncillo, Maya nut, Mongongo, Oak acorns, Ogbono nut, Paradise nut, Pili nut, Pine nut, Pistacchio, Pumpkin seed, water Caltrop; soybeans (Glycine sp., Glycine max);Lauraceae, for example avocado, Cinnamomum, camphor; Solanaceae sp. (for example tomatoes, potatoes, peppers, bell peppers, capsicum, aubergines, eggplant, tobacco), Rubiaceae sp. (for example coffee);
spices like Ajwain (Trachyspermum ammi), Allspice (Pimenta dioica), Alkanet (Anchusa arvensis), Amchur - mango powder (Mangifera), Angelica (Angelica archangelica), Anise (Pimpinella anisum), Aniseed myrtle (Syzygium anisatum), Annatto (Bixa orellana L.), Apple mint (Mentha suaveolens), Artemisia vulgaris/Mugwort, Asafoetida (Ferula assafoetida), Berberis, Banana, Basil (Ocimum basilicum), Bay leaves, Bistort (Persicaria bis-torta"), Black cardamom, Black cumin,

Blackcurrant, Black limes, Bladder wrack (Fucus vesiculosus), Blue Co-hosh, Blue-leaved Mallee (Eucalyptus polybractea), Bog Labrador Tea (Rhododendron groenlandicum), Boldo (Peumus boldus), Bolivian Coriander (Porophyllum ruderale), Borage (Borago officinalis), Calamus, Calendula, Calumba (Jateorhiza calumba), Chamomile, Candle nut, Cannabis, Caper (Capparis spinosa), Caraway, Cardamom, Carob Pod, Cassia, Casuarina, Catnip, Cat's Claw, Catsear, Cayenne pepper, Celastrus Paniculatus - Herb., Celery salt, Celery seed, Centaury, Chervil (Anthriscus cerefolium), Chickweed, Chicory, Chile pepper, Chili powder, Cinchona, Chives (Allium schoenoprasum), Cicely (Myrrhis odorata), Cilantro (see Coriander) (Coriandrum sativum), Cinnamon (and Cassia), Cinnamon Myrtle (Backhousia myrtifolia), Clary, Cleavers, Clover, Cloves, Coffee, Coltsfoot, Comfrey, Common Rue, Condurango, Coptis, Coriander, Costmary (Tanacetum balsamita), Couchgrass, Cow Parsley (Anthriscus sylvestris), Cowslip, Cramp Bark (Viburnum opulus), Cress, Cuban Oregano (Plectranthus amboinicus), Cudweed, Cumin, Curry leaf (Murraya koenigii), Damiana (Turnera aphrodisiaca, T. diffusa), Dandelion (Taraxacum officinale), Demulcent, Devil's claw (Harpagophytum procumbens), Dill seed, Dill (Anethum graveolens), Dorrigo Pepper (Tasmannia stipitata), Echinacea -, Echinopanax Elatum, Edelweiss, Elderberry, Elderflower, Elecampane, Eleutherococcus senticosus, Emmenagogue, Epazote (Chenopodium ambrosioides), Ephedra -, Eryngium foetidum, Eucalyptus, Fennel (Foeniculum vulgare), Fenugreek, Feverfew, Figwort, File powder, Five-spice powder (Chinese), Fo-ti-tieng, Fumitory, Galangal, Garam masala, Garden cress, Garlic chives, Garlic, Ginger (Zingiber officinale), Ginkgo biloba, Ginseng, Ginseng, Siberian (Eleutherococcus senticosus), Goat's Rue (Galega officinalis), Goada masala, Golden Rod, Golden Seal, Gotu Kola, Grains of paradise (Aframomum melegueta), Grains of Selim (Xylopia aethiopica), Grape seed extract, Green tea, Ground Ivy, Guaco, Gypsywort, Hawthorn (Crataegus sanguinea), Hawthorne Tree, Hemp, Herbes de Provence, Hibiscus, Holly, Holy Thistle, Hops, Horehound, Horseradish, Horsetail (Equisetum telmateia), Hyssop (Hyssopus officinalis), Jalap, Jasmine, Jiaogulan (Gynostemma pentaphyllum), Joe Pye weed (Gravelroot), John the Conqueror, Juniper, Kaffir Lime Leaves (Citrus hystrix, C. papedia), Kaala masala, Knotweed, Kokam, Labrador tea, Lady's Bedstraw, Lady's Mantle, Land cress, Lavender (Lavandula spp.), Ledum, Lemon Balm (Melissa Officinalis), Lemon basil, Lemongrass (Cymbopogon citratus, C. flexuosus, and other species), Lemon Ironbark (Eucalyptus staigeriana), Lemon mint, Lemon Myrtle (Backhousia citriodora), Lemon Thyme, Lemon verbena (Lippia citriodora), Licorice - adaptogen, Lime Flower, Limnophila aromatica, Lingzhi, Linseed, Liquorice, Long pepper, Lovage (Levisticum officinale), Luohanguo, Mace, Mahlab, Malabathrum, Manchurian Thorn Tree (Aralia manchurica)]], Mandrake, Marjoram (Origanum majorana), Marrubium vulgare, Marsh Labrador Tea, Marshmallow, Mastic, Meadowsweet, Mei Yen, Melegueta pepper (Aframomum melegueta), Mint (Mentha spp.), Milk thistle (Silybum), Bergamot (Monarda didyma), Motherwort, Mountain Skullcap, Mullein (Verbascum thapsus), Mustard, Mustard seed, Nashia inaguensis, Neem, Nepeta, Nettle, Nigella sativa, Nigella (Kolanji, Black caraway), Noni, Nutmeg (and Mace) Marijuana, Oenothera (Oenothera biennis et al), Olida (Eucalyptus olida), Oregano (Origanum vulgare, O. heracleoticum, and other species), Orris root, Osmorhiza, Olive Leaf (used in tea and as herbal supplement), Panax quinquefolius, Pandan leaf, Paprika, Parsley (Petroselinum crispum), Passion Flower, Patchouli, Pennyroyal, Pepper (black, white, and green), Peppermint, Peppermint Gum (Eucalyptus dives), Perilla, Plantain, Pomegranate, Ponch phoran, Poppy seed, Primrose (Primula) - candied flowers, tea, Psyllium, Purslane, Quassia, Quatre épices, Ramsons, Ras el-hanout, Raspberry (leaves), Reishi, Restharrow, Rhodiola rosea, Riberry (Syzygium luehmannii), Rocket/Arugula, Roman chamomile, Rooibos, Rosehips, Rosemary (Rosmarinus officinalis), Rowan Berries, Rue, Safflower, Saffron, Sage (Salvia officinalis), Saigon Cinnamon, St John's Wort, Salad Burnet (Sanguisorba minor or Poterium sanguisorba), Salvia, Sichuan Pepper (Sansho), Sassafras, Savory (Satureja hortensis, S. Montana), Schisandra (Schisandra chinensis), Scutellaria costaricana, Senna (herb), Senna obtusifolia, Sesame seed, Sheep Sorrel, Shepherd's Purse, Sialagogue, Siberian Chaga, Siberian ginseng (Eleutherococcus senticosus), Siraitia grosvenorii (luohanguo), Skullcap, Sloe Berries, Smudge Stick, Sonchus, Sorrel (Rumex spp.), Southernwood, Spearmint, Speedwell, Squill, Star anise, Stevia, Strawberry Leaves, Suma (Pfaffia paniculata), Sumac, Summer savory, Sutherlandia frutescens, Sweet grass, Sweet cicely (Myrrhis odorata), Sweet woodruff, Szechuan pepper (Xanthoxylum piperitum), Tacamahac, Tamarind, Tandoori masala, Tansy, Tarragon (Artemisia dracunculus), Tea, Teucrium polium, Thai basil, Thistle, Thyme, Toor Dall, Tormentil, Tribulus terrestris, Tulsi (Ocimum tenuiflorum), Turmeric (Curcuma longa), Uva Ursi also known as Bearberry, Vanilla (Vanilla planifolia), Vasaka, Vervain, Vetiver, Vietnamese Coriander (Persicaria odorata), Wasabi (Wasabia japonica), Watercress, Wattleseed, Wild ginger, Wild Lettuce, Wild thyme, Winter savory, Witch Hazel, Wolfberry, Wood Avens, Wood Betony, Woodruff, Wormwood, Yarrow, Yerba Buena, Yohimbe, Za'atar, Zedoary Root.

Stevia rebaudiana;

Theobroma sp. (for example Theobroma cacao: cocoa)

vegetables, for example spinach, lettuce, Asparagaceae (e.g. asparagus), Cruciferae sp. (e.g. white cabbage, red cabbage, broccoli, cauliflower, Brussels sprouts, pak choi, kohlrabi, radishes, horseradish, cress and chinese cabbage), onions, bell peppers, artichokes and chicory - including root chicory, endive or common chicory, leeks and onions;

Umbelliferae sp. (e.g. carrots, parsley, celery and celeriac);

Vitis sp. (for example Vitis vinifera: grape vine, raisins, table grapes)

or else plants such as turf, sugar cane, tea (Camellia sinensis), hops, and ornamentals, for example flowers, shrubs, deciduous trees and coniferous trees. This enumeration is no limitation.

The following plants are considered to be particularly suitable target crops: cotton, aubergine, turf, pome fruit, stone fruit, soft fruit, maize, wheat, barley, cucumber, tobacco, vines, rice, cereals, pear, beans, soybeans, oilseed rape, tomato, bell pepper, melons, cabbage, potato and apple.
Examples of trees are: Abies sp., Eucalyptus sp., Picea sp., Pinus sp., Aesculus sp., Platanus sp., Tilia sp., Acer sp., Tsuga sp., Fraxinus sp., Sorbus sp., Betula sp., Crataegus sp., Ulmus sp., Quercus sp., Fagus sp., Salix sp., Populus sp..
Examples of turf grasses are including cool-season turf grasses and warm-season turf grasses.
Cold-season turf grasses are bluegrasses (Poa spp.), such as Kentucky bluegrass (Poa pratensis L.), rough bluegrass (Poa trivialis L.), Canada bluegrass (Poa compressa L.), annual bluegrass (Poa annua L.), upland bluegrass (Poa glaucantha Gaudin), wood bluegrass (Poa nemoralis L.) and bulbous bluegrass (Poa bulbosa L.); bentgrasses (Agrostis spp.) such as creeping bentgrass (Agrostis palustris Huds.), colonial bentgrass (Agrostis tenuis Sibth.), velvet bentgrass (Agrostis canina L.), South German mixed bentgrass (Agrostis spp. including Agrostis tenuis Sibth., Agrostis canina L., and Agrostis palustris Huds.), and redtop (Agrostis alba L.);
fescues (Festuca spp.), such as red fescue (Festuca rubra L. spp. rubra), creeping fescue (Festuca rubra L.), chewings fescue (Festuca rubra commutata Gaud.), sheep fescue (Festuca ovina L.), hard fescue (Festuca longifolia Thuill.), hair fescue (Festucu capillata Lam.), tall fescue (Festuca arundinacea Schreb.) and meadow fescue (Festuca elanor L.);
ryegrasses (Lolium spp.), such as annual ryegrass (Lolium multiflorum Lam.), perennial ryegrass (Lolium perenne L.) and Italian ryegrass (Lolium multiflorum Lam.);
and wheatgrasses (Agropyron spp.), such as fairway wheatgrass (Agropyron cristatum (L.) Gaertn.), crested wheatgrass (Agropyron desertorum (Fisch.) Schult.) and western wheatgrass (Agropyron smithii Rydb.);
and further cool-season turf grasses like beachgrass (Ammophila breviligulata Fern.), smooth bromegrass (Bromus inermis Leyss.), cattails such as timothy (Phleum pratense L.), sand cattail (Phleum subulatum L.), orchardgrass (Dactylis glomerata L.), weeping alkaligrass (Puccinellia distans (L.) Parl.) and crested dog's-tail (Cynosurus cristatus L.).
Warm-season turf grasses are Bermuda grass (Cynodon spp. L. C. Rich), zoysia grass (Zoysia spp. Willd.), St. Augustine grass (Stenotaphrum secundatum Walt Kuntze), centipede grass (Eremochloa ophiuroides Munro Hack.), carpetgrass (Axonopus affinis Chase), Bahia grass (Paspalum notatum Flugge), Kikuyu grass (Pennisetum clandestinum Hochst. ex Chiov.), buffalo grass (Buchloe dactyloids (Nutt.) Engelm.), blue grama (Bouteloua gracilis (H.B.K.) Lag. ex Griffiths), seashore paspalum (Paspalum vaginatum Swartz) and sideoats grama (Bouteloua curtipendula (Michx. Torr.).
In particular, the compositions according to the invention are suitable for controlling the following plant diseases:

*Albugo* spp. (white rust) on ornamental plants, vegetable crops (e.g. *A. candida*) and sunflowers (e.g. *A. tragopogonis*); *Alternaria* spp. (black spot disease, black blotch) on vegetables, oilseed rape (e.g. *A. brassicola* or *A. brassicae*), sugar beet (e.g. *A. tenuis*), fruit, rice, soybeans and also on potatoes (e.g. *A. solani* or *A. alternata*) and tomatoes (e.g. *A. solani* or *A. alternata*) *and Alternaria* spp. (black head) on wheat; *Aphanomyces* spp. on sugar beet and vegetables; *Ascochyta* spp. on cereals and vegetables, e.g. *A. tritici* (Ascochyta leaf blight) on wheat and *A. hordei* on barley; *Bipolaris* and *Drechslera* spp. (teleomorph: *Cochliobolus* spp.), e.g. leaf spot diseases (*D. maydis* and *B. zeicola*) on corn, e.g. glume blotch (*B. sorokiniana*) on cereals and e.g. *B. oryzae* on rice and on lawn; *Blumeria* (old name: *Erysiphe*) *graminis* (powdery mildew) on cereals (e.g. wheat or barley); *Botryosphaeria* spp. ('Slack Dead Arm Disease') on grapevines (e.g. *B. obtusa*); *Botrytis cinerea* (teleomorph: *Botryotinia fuckeliana:* gray mold, gray rot) on soft fruit and pomaceous fruit (inter alia strawberries), vegetables (inter alia lettuce, carrots, celeriac and cabbage), oilseed rape, flowers, grapevines, forest crops and wheat (ear mold); *Bremia lactucae* (downy mildew) on lettuce; *Ceratocystis* (syn. *Ophiostoma*) spp. (blue stain fungus) on deciduous trees and coniferous trees, e.g. *C. ulmi* (Dutch elm disease) on elms; *Cercospora* spp. (Cereospora leat spot) on corn (e.g. *C. zeaemaydis*), rice, sugar beet (e.g. *C beticola*), sugar cane, vegetables, coffee, soybeans (e.g. *C. sojina* or *C. kikuchil*) and rice; *Cladosporium* spp. on tomato (e.g. *C. fulvum:* tomato leaf mold) and cereals, e.g. *C. herbarum* (ear rot) on wheat; *Claviceps purpurea* (ergot) on cereals; *Cochliobolus* (anamorph: *Helminthosporium* or *Bipolaris*) spp. (leaf spot) on corn (e.g. *C. carbonum*), cereals (e.g. C. *sativus,* anamorph: *B. sorokiniana:* glume blotch) and rice (tor example *C. miyabeanus,* anamorph: *H. oryzae*); *Colletotrichum*(teleomorph: *Glomerella*) spp. (anthracnosis) on cotton (e.g. *C. gossypii*), corn (e.g. *C. graminicola:* stem rot and anthracnosis), soft fruit, potatoes (e.g. *C. coccodes:* wilt disease), beans (e.g. *C. lindemuthianum*) and soybeans (e.g. *C. truncatum*); *Corticium* spp., e.g. *C. sasakii* (sheath blight) on rice; *Corynespora cassiicola* (leaf spot) on soybeans and ornamental plants; *Cycloconium* spp., e.g. *C. oleaginum* on olives; *Cylindrocarpon* spp. (e.g. fruit tree cancer or black foot disease of grapevine, teleomorph: *Nectria* or *Neonectria* spp.) on fruit trees, grapevines (e.g. *C. liriodendn;* teleomorph: *Neonectria liriodendri,* black foot disease) and many ornamental trees; *Dematophora* (teleomorph: *Rosellinia*) *necatrix* (root/stem rot) on soybeans; *Diaporthe* spp. e.g. *D. phaseolorum* (stem disease) on soybeans; *Drechslera* (syn. *Helminthosporium,* teleomorph: *Pyrenophora*) spp. on corn, cereals, such as barley (e.g. *D. teres,* net blotch) and on wheat (e.g. *D. tritici-repentis:* DTR leaf spot), rice and lawn; Esca disease (dieback of grapevine, apoplexia) on grapevines, caused by *Formitiporia* (syn. *Phellinus*) *punctata, F mediterranea. Phaeomoniella chlamydospora* (old name *Phaeoacremonium chlamydosporum*), *Phaeoacremonium aleophilum* and/or *Botryosphaeria obtusa; Elsinoe* spp. on

pome fruit (*E. pyri*) and soft fruit (*E. veneta:* anthracnosis) and also grapevines (*E. ampelina:* anthracnosis); *Entyloma oryzae* (leaf smut) on rice; *Epicoccum* spp. (black head) on wheat; *Erysiphe* spp. (powdery mildew) on sugar beet (*E. betae*), vegetables (e.g. *E. pisi*), such as cucumber species (e.g. *E. cichoracearum*) and cabbage species, such as oilseed rape (e.g. *E. cruciferarum*)*; Eutypa fata* (Eutypa cancer or dieback, anamorph: *Cytosporina lata,* syn. *Libertella blepharis*) on fruit trees, grapevines and many ornamental trees; *Exserohilum* (syn. *Helminthosporium*) spp. on corn (e.g. *E. turcicum*); *Fusarium* (teleomorph: *Gibberella*) spp. (wilt disease, root and stem rot) on various plants, such as *e.g. F. graminearum* or *F. culmorum* (root rot and silver-top) on cereals (e.g. wheat or barley), *F. oxysporum* on tomatoes, *F. solani* on soybeans and *F. verticillioides* on corn; *Gaeumannomyces graminis* (takeall) on cereals (e.g. wheat or barley) and corn; *Gibberella* spp. on cereals (e.g. *G. zeae*) and rice (e.g. *G. fujikuroi:* bakanae disease); *Glomerella cingulata* on grapevines, pomaceous fruit and other plants and *G. gossypii* on cotton; *grainstaining complex* on rice; *Guignardia bidwellii* (*black rot*) on grapevines; *Gymnosporangium* spp. on Rosaceae and juniper, e.g. *G. sabinae* (pear rust) on pears; *Helminthosporium* spp. (syn. *Drechslera,* teleomorph: *Cochliobolus*) on corn, cereals and rice; *Hemileia* spp., e.g. *H. vastatrix* (coffee leaf rust) on coffee; *Isariopsis clavispora* (syn. *Cladosporium vitis*) on grapevines; *Macrophomina phaseolina* (syn. *phaseoli*) (root/stem rot) on soybeans and cotton; *Microdochium* (syn. *Fusarium*) *nivale* (pink snow mold) on cereals (e.g. wheat or barley); *Microsphaera diffusa* (powdery mildew) on soybeans; *Monilinia* spp., e.g. *M. laxa. M. fructicola* and *M. fructigena* (blossom and twig blight) on stone fruit and other Rosaceae; *Mycosphaerella* spp. on cereals, bananas, soft fruit and peanuts, such as e.g. *M. graminicola* (anamorph: *Septoria tritici,* Septoria leaf blotch) on wheat or *M. fijiensis* (Sigatoka disease) on bananas; *Peronospora* spp. (downy mildew) on cabbage (e.g. *P. brassicae*), oilseed rape (e.g. *P. parasitica*), bulbous plants (e.g. *P. destructor*), tobacco (*P. tabacina*) and soybeans (e.g. *P. manshurica*); *Phakopsora pachyrhizi* and *P. meibomiae* (*soybean rust*) on soybeans; *Phialophora* spp. e.g. on grapevines (e.g. *P. tracheiphila* and *P. tetraspora*) and soybeans (e.g. *P. gregata:* stem disease); *Phoma lingam* (root and stem rot) on oilseed rape and cabbage and *P. betae* (leaf spot) on sugar beet; *Phomopsis* spp. on sunflowers, grapevines (e.g. *P. viticola:* dead-arm disease) and soybeans (e.g. stem canker/stem blight: *P. phaseoli,* teleomorph: *Diaporthe phaseolorum*); *Physoderma maydis* (brown spot) on corn; *Phytophthora* spp. (wilt disease, root, leaf, stem and fruit rot) on various plants, such as on bell peppers and cucumber species (e.g. *P. capsici*), soybeans (e.g. *P. megasperma,* syn. *P. sojae*), potatoes and tomatoes (e.g. *P. infestans.* late blight and brown rot) and deciduous trees (e.g. *P. ramorum* sudden oak death); *Plasmodiophora brassicae* (club-root) on cabbage, oilseed rape, radish and other plants; *Plasmopara* spp., e.g. *P. viticola* (peronospora of grapevines, downy mildew) on grapevines and *P. halstedii* on sunflowers; *Podosphaera* spp. (powdery mildew) on Rosaceae, hops, pomaceaus fruit and soft fruit, e.g. *P. leucotricha* on apple; *Polymyxa* spp., e.g. on cereals, such as barley and wheat (*P. graminis*) and sugar beet (*P. betae*) and the viral diseases transmitted thereby; *Pseudocercosporella herpotrichoides* (eyespot/stem break, teleomorph: *Tapesia yallundae*) on cereals. e.g. wheat or barley; *Pseudoperonospora* (downy mildew) on various plants, e.g. *P. cubensis* on cucumber species or *P. humili* on hops; *Pseudopezicula tracheiphila* (angular leaf scorch, anamorph *Phialophora*) on grapevines; *Puccinia* spp. (rust disease) on various plants, e.g. *P. triticina* (brown rust of wheat), *P. striiformis* (yellow rust). *P. hordei* (dwarf leaf rust), *P. graminis* (black rust) or *P. recondita* (brown rust of rye) on cereals, such as e.g. wheat, barley or rye. *P. kuehnii* on sugar cane and, e.g., on asparagus (e.g. *P. asparagi*); *Pyrenophora* (anamorph: *Drechslera*) *tritici-repentis* (speckled leaf blotch) on wheat or *P. teres* (net blotch) on barley; *Pyricularia* spp., e.g. *P. oryzae* (teleomorph: *Magnaporthe grisea.* rice blast) on rice and *P. grisea* on lawn and cereals; *Pythium* spp. (*damping-off disease*) on lawn, rice, corn, wheat, cotton, oilseed rape, sunflowers, sugar beet, vegetables and other plants (e.g. *P. ultimum* or *P. aphanidermatum*); *Ramularia* spp., e.g. *R. collocygni*(*Ramularia* leaf and lawn spot/physiological leaf spot) on barley and *R. beticola* on sugar beet; *Rhizoctonia spp.* on cotton, rice, potatoes, lawn, corn, oilseed rape, potatoes, sugar beet, vegetables and on various other plants, for example *R. solani* (root and stern rot) on soybeans, *R. solani* (sheath blight) on rice or *R. cerealis* (sharp eyespot) on wheat or barley; *Rhizopus stolonifer* (soft rot) on strawberries, carrots, cabbage, grapevines and tomato; *Rhynchosporium secalis* (leaf spot) on barley, rye and triticale; *Sarocladium oryzae* and *S. attenuatum* (sheath rot) on rice; *Sclerotinia* spp. (stem or white rot) on vegetable and field crops, such as oilseed rape, sunflowers (e.g. *Sclerotinia sclerotiorum*) and soybeans (e.g. *S. rolfsii*), · *Septoria* spp. on various plants, e.g. *S. glycines* (leaf spot) on soybeans, *S. tritici* (*Septoria* leaf blotch) on wheat and *S.* (syn. *Stagonospora*) *nodorum* (leaf blotch and glume blotch) on cereals; *Uncinula* (syn. *Erysiphe*) *necator* (*powdery* mildew, anamorph: *Oidium tuckeri*) on grapevines; *Setospaeria* spp. (leaf spot) on corn (e.g. *S. turcicum,* syn. *Helminthosporium turcicum*) and lawn; *Sphacelotheca* spp. (head smut) on corn, (e.g. *S. reiliana:* kernel smut), millet and sugar cane; *Sphaerotheca fuliginea* (powdery mildew) on cucumber species; *Spongospora subterranea* (powdery scab) on potatoes and the viral diseases transmitted thereby; *Stagonospora* spp. on cereals, e.g. *S. nodorum* (leaf blotch and glume blotch, teleomorph: *Leptosphaeria* [syn. *Phaeosphaeria] nodorum*) on wheat; *Synchytrium endobioticum* on potatoes (potato wart disease); *Taphrina* spp., e.g. *T. deformans* (curly-leaf disease) on peach and *T. pruni* (plum-pocket disease) on pi ums; *Thielaviopsis* spp. (black root rot) on tobacco, pome fruit, vegetable crops, soybeans and cotton, e.g. *T. basicola* (syn. *Chalara elegans*); *Tilletia* spp. (*bunt or stinking smut*) on cereals, such as e.g. *T. tritici* (syn. *T. caries,* wheat bunt) and *T. controversa*

(dwarf bunt) on wheat; *Typhula incarnata* (gray snow mold) on barley or wheat; *Urocystis* spp., e.g. *U. occulta* (flag smut) on rye; *Uromyces* spp. (rust) on vegetable plants, such as beans (e.g. *U. appendiculatus,* syn. *U. phaseoli*) and sugar beet (e.g. *U. betae*); *Ustilago* spp. (loose smut) on cereals (e.g. *U. nuda* and *U. avaenae*), corn (e.g. *U. maydis:* corn smut) and sugar cane; *Venturia* spp. (scab) on apples (e.g. *V. inaequalis*) and pears and *Verticillium* spp. (leaf and shoot wilt) on various plants, such as fruit trees and ornamental trees, grapevines, soft fruit, vegetable and field crops, such as e.g. *V. dahliae* on strawberries, oilseed rape, potatoes and tomatoes.

*Plant Growth Regulation*

In some cases, the inventive compounds can, at particular concentrations or application rates, also be used as herbicides, safeners, growth regulators or agents to improve plant properties, or as microbicides, for example as fungicides, antimycotics, bactericides, viricides (including compositions against viroids) or as compositions against MLO (Mycoplasma-like organisms) and RLO (Rickettsia-like organisms). If appropriate, they can also be used as intermediates or precursors for the synthesis of other active ingredients.

The compositions according to the invention intervene in the metabolism of the plants and can therefore also be used as growth regulators.

Plant growth regulators may exert various effects on plants. The effect of the substances depends essentially on the time of application in relation to the developmental stage of the plant, and also on the amounts of active ingredient applied to the plants or their environment and on the type of application. In each case, growth regulators should have a particular desired effect on the crop plants.

Plant growth-regulating compounds can be used, for example, to inhibit the vegetative growth of the plants. Such inhibition of growth is of economic interest, for example, in the case of grasses, since it is thus possible to reduce the frequency of grass cutting in ornamental gardens, parks and sport facilities, on roadsides, at airports or in fruit crops. Also of significance is the inhibition of the growth of herbaceous and woody plants on roadsides and in the vicinity of pipelines or overhead cables, or quite generally in areas where vigorous plant growth is unwanted.

Also important is the use of growth regulators for inhibition of the longitudinal growth of cereal. This reduces or completely eliminates the risk of lodging of the plants prior to harvest. In addition, growth regulators in the case of cereals can strengthen the culm, which also counteracts lodging. The employment of growth regulators for shortening and strengthening culms allows the deployment of higher fertilizer volumes to increase the yield, without any risk of lodging of the cereal crop.

In many crop plants, inhibition of vegetative growth allows denser planting, and it is thus possible to achieve higher yields based on the soil surface. Another advantage of the smaller plants obtained in this way is that the crop is easier to cultivate and harvest.

Inhibition of the vegetative plant growth may also lead to enhanced yields because the nutrients and assimilates are of more benefit to flower and fruit formation than to the vegetative parts of the plants.

Frequently, growth regulators can also be used to promote vegetative growth. This is of great benefit when harvesting the vegetative plant parts. However, promoting vegetative growth may also promote generative growth in that more assimilates are formed, resulting in more or larger fruits.

In some cases, yield increases may be achieved by manipulating the metabolism of the plant, without any detectable changes in vegetative growth. In addition, growth regulators can be used to alter the composition of the plants, which in turn may result in an improvement in quality of the harvested products. For example, it is possible to increase the sugar content in sugar beet, sugar cane, pineapples and in citrus fruit, or to increase the protein content in soya or cereals. It is also possible, for example, to use growth regulators to inhibit the degradation of desirable ingredients, for example sugar in sugar beet or sugar cane, before or after harvest. It is also possible to positively influence the production or the elimination of secondary plant ingredients. One example is the stimulation of the flow of latex in rubber trees.

Under the influence of growth regulators, parthenocarpic fruits may be formed. In addition, it is possible to influence the sex of the flowers. It is also possible to produce sterile pollen, which is of great importance in the breeding and production of hybrid seed.

Use of growth regulators can control the branching of the plants. On the one hand, by breaking apical dominance, it is possible to promote the development of side shoots, which may be highly desirable particularly in the cultivation of ornamental plants, also in combination with an inhibition of growth. On the other hand, however, it is also possible to inhibit the growth of the side shoots. This effect is of particular interest, for example, in the cultivation of tobacco or in the cultivation of tomatoes.

Under the influence of growth regulators, the amount of leaves on the plants can be controlled such that defoliation of the plants is achieved at a desired time. Such defoliation plays a major role in the mechanical harvesting of cotton, but is also of interest for facilitating harvesting in other crops, for example in viticulture. Defoliation of the plants can also be undertaken to lower the transpiration of the plants before they are transplanted.

Growth regulators can likewise be used to regulate fruit dehiscence. On the one hand, it is possible to prevent

premature fruit dehiscence. On the other hand, it is also possible to promote fruit dehiscence or even flower abortion to achieve a desired mass ("thinning"), in order to eliminate alternation. Alternation is understood to mean the characteristic of some fruit species, for endogenous reasons, to deliver very different yields from year to year. Finally, it is possible to use growth regulators at the time of harvest to reduce the forces required to detach the fruits, in order to allow mechanical harvesting or to facilitate manual harvesting.

Growth regulators can also be used to achieve faster or else delayed ripening of the harvested material before or after harvest. This is particularly advantageous as it allows optimal adjustment to the requirements of the market. Moreover, growth regulators in some cases can improve the fruit colour. In addition, growth regulators can also be used to concentrate maturation within a certain period of time. This establishes the prerequisites for complete mechanical or manual harvesting in a single operation, for example in the case of tobacco, tomatoes or coffee.

By using growth regulators, it is additionally possible to influence the resting of seed or buds of the plants, such that plants such as pineapple or ornamental plants in nurseries, for example, germinate, sprout or flower at a time when they are normally not inclined to do so. In areas where there is a risk of frost, it may be desirable to delay budding or germination of seeds with the aid of growth regulators, in order to avoid damage resulting from late frosts.

Finally, growth regulators can induce resistance of the plants to frost, drought or high salinity of the soil. This allows the cultivation of plants in regions which are normally unsuitable for this purpose.

*Resistance Induction / Plant Health and other effects*

The active compounds according to the invention also exhibit a potent strengthening effect in plants. Accordingly, they can be used for mobilizing the defences of the plant against attack by undesirable microorganisms.

Plant-strengthening (resistance-inducing) substances are to be understood as meaning, in the present context, those substances which are capable of stimulating the defence system of plants in such a way that the treated plants, when subsequently inoculated with undesirable microorganisms, develop a high degree of resistance to these microorganisms.

The active compounds according to the invention are also suitable for increasing the yield of crops. In addition, they show reduced toxicity and are well tolerated by plants.

Further, in context with the present invention plant physiology effects comprise the following:

Abiotic stress tolerance, comprising temperature tolerance, drought tolerance and recovery after drought stress, water use efficiency (correlating to reduced water consumption), flood tolerance, ozone stress and UV tolerance, tolerance towards chemicals like heavy metals, salts, pesticides (safener) etc..

Biotic stress tolerance, comprising increased fungal resistance and increased resistance against nematodes, viruses and bacteria. In context with the present invention, biotic stress tolerance preferably comprises increased fungal resistance and increased resistance against nematodes

Increased plant vigor, comprising plant health / plant quality and seed vigor, reduced stand failure, improved appearance, increased recovery, improved greening effect and improved photosynthetic efficiency.

Effects on plant hormones and/or functional enzymes.

Effects on growth regulators (promoters), comprising earlier germination, better emergence, more developed root system and/or improved root growth, increased ability of tillering, more productive tillers, earlier flowering, increased plant height and/or biomass, shorting of stems, improvements in shoot growth, number of kernels/ear, number of ears/$m^2$, number of stolons and/or number of flowers, enhanced harvest index, bigger leaves, less dead basal leaves, improved phyllotaxy, earlier maturation / earlier fruit finish, homogenous riping, increased duration of grain filling, better fruit finish, bigger fruit/vegetable size, sprouting resistance and reduced lodging.

Increased yield, referring to total biomass per hectare, yield per hectare, kernel/fruit weight, seed size and/or hectolitre weight as well as to increased product quality, comprising:

improved processability relating to size distribution (kernel, fruit, etc.), homogenous riping, grain moisture, better milling, better vinification, better brewing, increased juice yield, harvestability, digestibility, sedimentation value, falling number, pod stability, storage stability, improved fiber length/strength/uniformity, increase of milk and/or meet quality of silage fed animals, adaption to cooking and frying;

further comprising improved marketability relating to improved fruit/grain quality, size distribution (kernel, fruit, etc.), increased storage / shelf-life, firmness / softness, taste (aroma, texture, etc.), grade (size, shape, number of berries, etc.), number of berries/fruits per bunch, crispness, freshness, coverage with wax, frequency of physiological disorders, colour, etc.;

further comprising increased desired ingredients such as e.g. protein content, fatty acids, oil content, oil quality, aminoacid composition, sugar content, acid content (pH), sugar/acid ratio (Brix), polyphenols, starch content, nutritional quality, gluten content/index, energy content, taste, etc.;

and further comprising decreased undesired ingredients such as e.g. less mycotoxines, less aflatoxines, geosmin level, phenolic aromas, lacchase, polyphenol oxidases and peroxidases, nitrate content etc.

Sustainable agriculture, comprising nutrient use efficiency, especially nitrogen (N)-use efficiency, phosphours (P)-use efficiency, water use efficiency, improved transpiration, respiration and/or $CO_2$ assimilation rate, better nodulation, improved Ca-metabolism etc..

Delayed senescence, comprising improvement of plant physiology which is manifested, for example, in a longer grain filling phase, leading to higher yield, a longer duration of green leaf colouration of the plant and thus comprising colour (greening), water content, dryness etc.. Accordingly, in the context of the present invention, it has been found that the specific inventive application of the compositions according to the invention makes it possible to prolong the green leaf area duration, which delays the maturation (senescence) of the plant. The main advantage to the farmer is a longer grain filling phase leading to higher yield. There is also an advantage to the farmer on the basis of greater flexibility in the harvesting time.

Therein "sedimentation value" is a measure for protein quality and describes according to Zeleny (Zeleny value) the degree of sedimentation of flour suspended in a lactic acid solution during a standard time interval. This is taken as a measure of the baking quality. Swelling of the gluten fraction of flour in lactic acid solution affects the rate of sedimentation of a flour suspension. Both a higher gluten content and a better gluten quality give rise to slower sedimentation and higher Zeleny test values. The sedimentation value of flour depends on the wheat protein composition and is mostly correlated to the protein content, the wheat hardness, and the volume of pan and hearth loaves. A stronger correlation between loaf volume and Zeleny sedimentation volume compared to SDS sedimentation volume could be due to the protein content influencing both the volume and Zeleny value (Czech J. Food Sci. Vol. 21, No. 3: 91-96, 2000).

Further the "falling number" as mentioned herein is a measure for the baking quality of cereals, especially of wheat. The falling number test indicates that sprout damage may have occurred. It means that changes to the physical properties of the starch portion of the wheat kernel has already happened. Therein, the falling number instrument analyzes viscosity by measuring the resistance of a flour and water paste to a falling plunger. The time (in seconds) for this to happen is known as the falling number. The falling number results are recorded as an index of enzyme activity in a wheat or flour sample and results are expressed in time as seconds. A high falling number (for example, above 300 seconds) indicates minimal enzyme activity and sound quality wheat or flour. A low falling number (for example, below 250 seconds) indicates substantial enzyme activity and sprout-damaged wheat or flour.

The term "more developed root system" / "improved root growth" refers to longer root system, deeper root growth, faster root growth, higher root dry/fresh weight, higher root volume, larger root surface area, bigger root diameter, higher root stability, more root branching, higher number of root hairs, and/or more root tips and can be measured by analyzing the root architecture with suitable methodologies and Image analysis programmes (e.g. WinRhizo).

The term "crop water use efficiency" refers technically to the mass of agriculture produce per unit water consumed and economically to the value of product(s) produced per unit water volume consumed and can e.g. be measured in terms of yield per ha, biomass of the plants, thousand-kernel mass, and the number of ears per m2.

The term "nitrogen-use efficiency" refers technically to the mass of agriculture produce per unit nitrogen consumed and economically to the value of product(s) produced per unit nitrogen consumed, reflecting uptake and utilization efficiency.

Improvement in greening / improved colour and improved photosynthetic efficiency as well as the delay of senescence can be measured with well-known techniques such as a HandyPea system (Hansatech). Fv/Fm is a parameter widely used to indicate the maximum quantum efficiency of photosystem II (PSII). This parameter is widely considered to be a selective indication of plant photosynthetic performance with healthy samples typically achieving a maximum Fv/Fm value of approx. 0.85. Values lower than this will be observed if a sample has been exposed to some type of biotic or abiotic stress factor which has reduced the capacity for photochemical quenching of energy within PSII. Fv/Fm is presented as a ratio of variable fluorescence (Fv) over the maximum fluorescence value (Fm). The Performance Index is essentially an indicator of sample vitality. (See e.g. *A*dvanced Techniques in Soil Microbiology, 2007, 11, 319-341; Applied Soil Ecology, 2000, 15, 169-182.)

The improvement in greening / improved colour and improved photosynthetic efficiency as well as the delay of senescence can also be assessed by measurement of the net photosynthetic rate (Pn), measurement of the chlorophyll content, e.g. by the pigment extraction method of Ziegler and Ehle, measurement of the photochemical efficiency (Fv/Fm ratio), determination of shoot growth and final root and/or canopy biomass, determination of tiller density as well as of root mortality.

Within the context of the present invention preference is given to improving plant physiology effects which are selected from the group comprising: enhanced root growth / more developed root system, improved greening, improved water use efficiency (correlating to reduced water consumption), improved nutrient use efficiency, comprising especially

improved nitrogen (N)-use efficiency, delayed senescence and enhanced yield.

Within the enhancement of yield preference is given as to an improvement in the sedimentation value and the falling number as well as to the improvement of the protein and sugar content - especially with plants selected from the group of cereals (preferably wheat).

Preferably the use of the compositions of the present invention relates to a combined use of a) preventively and/or curatively controlling unwanted microorganisms and b) at least one of enhanced root growth, improved greening, improved water use efficiency, delayed senescence and enhanced yield. From group b) enhancement of root system, water use efficiency and N-use efficiency is particularly preferred.

*Seed Treatment*

The invention further comprises a method for treating seed.

The invention further relates to seed which has been treated by one of the methods described in the previous paragraph. The inventive seeds are employed in methods for the protection of seed from unwanted microorganisms. In these methods, seed treated with at least one inventive active ingredient is used.

The compositions according to the invention are also suitable for treating seed. A large part of the damage to crop plants caused by harmful organisms is triggered by the infection of the seed during storage or after sowing, and also during and after germination of the plant. This phase is particularly critical since the roots and shoots of the growing plant are particularly sensitive, and even minor damage may result in the death of the plant. There is therefore a great interest in protecting the seed and the germinating plant by using appropriate compositions.

The control of unwanted microorganisms by treating the seed of plants has been known for a long time and is the subject of constant improvements. However, the treatment of seed entails a series of problems which cannot always be solved in a satisfactory manner. For instance, it is desirable to develop methods for protecting the seed and the germinating plant, which dispense with, or at least significantly reduce, the additional deployment of crop protection compositions after planting or after emergence of the plants. It is also desirable to optimize the amount of the active ingredient used so as to provide the best possible protection for the seed and the germinating plant from attack by unwanted microorganisms, but without damaging the plant itself by the active ingredient employed. In particular, methods for the treatment of seed should also take account of the intrinsic fungicidal properties of transgenic plants in order to achieve optimal protection of the seed and the germinating plant with a minimum expenditure of crop protection compositions.

The present invention therefore also relates to a method for protection of seed and germinating plants from attack by unwanted microorganisms, by treating the seed with an inventive composition. The invention likewise relates to the use of the inventive compositions for treatment of seed to protect the seed and the germinating plant from unwanted microorganisms. The invention further relates to seed which has been treated with an inventive composition for protection from unwanted microorganisms.

The control of unwanted microorganisms which damage plants post-emergence is effected primarily by treating the soil and the above-ground parts of plants with crop protection compositions. Owing to the concerns regarding a possible influence of the crop protection compositions on the environment and the health of humans and animals, there are efforts to reduce the amount of active ingredients deployed.

One of the advantages of the present invention is that the particular systemic properties of the compositions according to the invention mean that treatment of the seed with these compositions not only protects the seed itself, but also the resulting plants after emergence, from unwanted microorganisms. In this way, the immediate treatment of the crop at the time of sowing or shortly thereafter can be dispensed with.

It is likewise considered to be advantageous that the compositions according to the invention can especially also be used with transgenic seed, in which case the plant growing from this seed is capable of expressing a protein which acts against pests. By virtue of the treatment of such seed with the compositions according to the invention or compositions, merely the expression of the protein, for example an insecticidal protein, can control certain pests. Surprisingly, a further synergistic effect can be observed in this case, which additionally increases the effectiveness for protection against attack by pests.

The inventive compositions are suitable for protecting seed of any plant variety which is used in agriculture, in greenhouses, in forests or in horticulture and viticulture. In particular, this is the seed of cereals (such as wheat, barley, rye, triticale, sorghum/millet and oats), maize, cotton, soya beans, rice, potatoes, sunflower, bean, coffee, beet (for example sugar beet and fodder beet), peanut, oilseed rape, poppy, olive, coconut, cocoa, sugar cane, tobacco, vegetables (such as tomato, cucumbers, onions and lettuce), turf and ornamentals (see also below). The treatment of the seed of cereals (such as wheat, barley, rye, triticale and oats), maize and rice is of particular significance.

As also described below, the treatment of transgenic seed with the compositions according to the invention is of particular significance. This relates to the seed of plants containing at least one heterologous gene which enables the expression of a polypeptide or protein having insecticidal properties. The heterologous gene in transgenic seed can

originate, for example, from microorganisms of the species *Bacillus, Rhizobium, Pseudomonas, Serratia, Trichoderma, Clavibacter, Glomus* or *Gliocladium.* This heterologous gene preferably originates from *Bacillus* sp., in which case the gene product is effective against the European maize borer and/or the Western maize rootworm. The heterologous gene more preferably originates from *Bacillus thuringiensis.*

In the context of the present invention, the inventive composition is applied to the seed alone or in a suitable formulation. Preferably, the seed is treated in a state in which it is sufficiently stable for no damage to occur in the course of treatment. In general, the seed can be treated at any time between harvest and sowing. It is customary to use seed which has been separated from the plant and freed from cobs, shells, stalks, coats, hairs or the flesh of the fruits. For example, it is possible to use seed which has been harvested, cleaned and dried down to a moisture content of less than 15 % by weight. Alternatively, it is also possible to use seed which, after drying, for example, has been treated with water and then dried again.

When treating the seed, care must generally be taken that the amount of the inventive composition applied to the seed and/or the amount of further additives is selected such that the germination of the seed is not impaired, or that the resulting plant is not damaged. This has to be borne in mind in particular in the case of active ingredients which can have phytotoxic effects at certain application rates.

The inventive compositions can be applied directly, i.e. without containing any other components and without having been diluted. In general, it is preferable to apply the compositions to the seed in the form of a suitable formulation. Suitable formulations and methods for seed treatment are known to those skilled in the art and are described, for example, in the following documents: US 4,272,417, US 4,245,432, US 4,808,430, US 5,876,739, US 2003/0176428 A1, WO 2002/080675, WO 2002/028186.

The active ingredients usable in accordance with the invention can be converted to the customary seed dressing formulations, such as solutions, emulsions, suspensions, powders, foams, slurries or other coating compositions for seed, and also ULV formulations.

These formulations are prepared in a known manner, by mixing the active ingredients with customary additives, for example customary extenders and also solvents or diluents, dyes, wetting agents, dispersants, emulsifiers, antifoams, preservatives, secondary thickeners, adhesives, gibberellins and also water.

Useful dyes which may be present in the seed dressing formulations usable in accordance with the invention are all dyes which are customary for such purposes. It is possible to use either pigments, which are sparingly soluble in water, or dyes, which are soluble in water. Examples include the dyes known by the names Rhodamine B, C.I. Pigment Red 112 and C.I. Solvent Red 1.

Useful wetting agents which may be present in the seed dressing formulations usable in accordance with the invention are all substances which promote wetting and which are conventionally used for the formulation of active agrochemical ingredients. Preference is given to using alkyl naphthalenesulphonates, such as diisopropyl or diisobutyl naphthalenesulphonates.

Useful dispersants and/or emulsifiers which may be present in the seed dressing formulations usable in accordance with the invention are all nonionic, anionic and cationic dispersants conventionally used for the formulation of active agrochemical ingredients. Usable with preference are nonionic or anionic dispersants or mixtures of nonionic or anionic dispersants. Suitable nonionic dispersants include especially ethylene oxide/propylene oxide block polymers, alkylphenol polyglycol ethers and tristryrylphenol polyglycol ether, and the phosphated or sulphated derivatives thereof. Suitable anionic dispersants are especially lignosulphonates, polyacrylic acid salts and aryl-sulphonate/formaldehyde condensates.

Antifoams which may be present in the seed dressing formulations usable in accordance with the invention are all foam-inhibiting substances conventionally used for the formulation of active agrochemical ingredients. Silicone antifoams and magnesium stearate can be used with preference.

Preservatives which may be present in the seed dressing formulations usable in accordance with the invention are all substances usable for such purposes in agrochemical compositions. Examples include dichlorophene and benzyl alcohol hemiformal.

Secondary thickeners which may be present in the seed dressing formulations usable in accordance with the invention are all substances usable for such purposes in agrochemical compositions. Preferred examples include cellulose derivatives, acrylic acid derivatives, xanthan, modified clays and finely divided silica.

Adhesives which may be present in the seed dressing formulations usable in accordance with the invention are all customary binders usable in seed dressing products. Preferred examples include polyvinylpyrrolidone, polyvinyl acetate, polyvinyl alcohol and tylose.

The gibberellins which may be present in the seed dressing formulations usable in accordance with the invention may preferably be gibberellins A1, A3 (= gibberellic acid), A4 and A7; particular preference is given to using gibberellic acid. The gibberellins are known (cf. R. Wegler "Chemie der Pflanzenschutz- und Schadlingsbekampfungsmittel" [Chemistry of the Crop Protection Compositions and Pesticides], vol. 2, Springer Verlag, 1970, p. 401-412).

The seed dressing formulations usable in accordance with the invention can be used, either directly or after

previously having been diluted with water, for the treatment of a wide range of different seed, including the seed of transgenic plants. In this case, additional synergistic effects may also occur in interaction with the substances formed by expression.

For treatment of seed with the seed dressing formulations usable in accordance with the invention, or the preparations prepared therefrom by adding water, all mixing units usable customarily for the seed dressing are useful. Specifically, the procedure in the seed dressing is to place the seed into a mixer, to add the particular desired amount of seed dressing formulations, either as such or after prior dilution with water, and to mix everything until the formulation is distributed homogeneously on the seed. If appropriate, this is followed by a drying process.

*Mycotoxins*

In addition, the inventive treatment can reduce the mycotoxin content in the harvested material and the foods and feeds prepared therefrom. Mycotoxins include particularly, but not exclusively, the following: deoxynivalenol (DON), nivalenol, 15-Ac-DON, 3-Ac-DON, T2- and HT2-toxin, fumonisins, zearalenon, moniliformin, fusarin, diaceotoxyscirpenol (DAS), beauvericin, enniatin, fusaroproliferin, fusarenol, ochratoxins, patulin, ergot alkaloids and aflatoxins which can be produced, for example, by the following fungi: *Fusarium* spec., such as *F. acuminatum, F. asiaticum, F. avenaceum, F. crookwellense, F. culmorum, F. graminearum* (*Gibberella zeae*), *F. equiseti, F. fujikoroi, F. musarum, F. oxysporum, F. proliferatum, F. poae, F. pseudograminearum, F. sambucinum, F. scirpi, F. semitectum, F. solani, F. sporotrichoides, F. langsethiae, F. subglutinans, F. tricinctum, F. verticillioides* etc., and also by *Aspergillus* spec., such as *A. flavus, A. parasiticus, A. nomius, A. ochraceus, A. clavatus, A. terreus, A. versicolor, Penicillium* spec., such as *P. verrucosum, P. viridicatum, P. citrinum, P. expansum, P. claviforme, P. roqueforti, Claviceps* spec., such as *C. purpurea, C. fusiformis, C. paspali, C. africana, Stachybotrys* spec. and others.

*Material Protection*

The compositions according to the invention can also be used in the protection of materials, for protection of industrial materials against attack and destruction by unwanted microorganisms, for example fungi, and insects.

In addition, the inventive compositions can be used as antifouling compositions, alone or in combinations with other active ingredients.

Industrial materials in the present context are understood to mean inanimate materials which have been prepared for use in industry. For example, industrial materials which are to be protected by compositions according to the invention from microbial alteration or destruction may be adhesives, glues, paper, wallpaper and board/cardboard, textiles, carpets, leather, wood, fibers and tissues, paints and plastic articles, cooling lubricants and other materials which can be infected with or destroyed by microorganisms. Parts of production plants and buildings, for example cooling-water circuits, cooling and heating systems and ventilation and air-conditioning units, which may be impaired by the proliferation of microorganisms may also be mentioned within the scope of the materials to be protected. Industrial materials within the scope of the present invention preferably include adhesives, sizes, paper and card, leather, wood, paints, cooling lubricants and heat transfer fluids, more preferably wood.

The compositions according to the invention may prevent adverse effects, such as rotting, decay, discoloration, decoloration or formation of mould.

In the case of treatment of wood the compounds/compositions according to the invention may also be used against fungal diseases liable to grow on or inside timber. The term "timber" means all types of species of wood, and all types of working of this wood intended for construction, for example solid wood, high-density wood, laminated wood, and plywood. The method for treating timber according to the invention mainly consists in contacting one or more compounds according to the invention or a composition according to the invention; this includes for example direct application, spraying, dipping, injection or any other suitable means.

In addition, the inventive compounds can be used to protect objects which come into contact with saltwater or brackish water, especially hulls, screens, nets, buildings, moorings and signalling systems, from fouling.

The inventive method for controlling unwanted microorganisms can also be employed for protecting storage goods. Storage goods are understood to mean natural substances of vegetable or animal origin or processed products thereof which are of natural origin, and for which long-term protection is desired. Storage goods of vegetable origin, for example plants or plant parts, such as stems, leaves, tubers, seeds, fruits, grains, can be protected freshly harvested or after processing by (pre)drying, moistening, comminuting, grinding, pressing or roasting. Storage goods also include timber, both unprocessed, such as construction timber, electricity poles and barriers, or in the form of finished products, such as furniture. Storage goods of animal origin are, for example, hides, leather, furs and hairs. The compositions according to the invention may prevent adverse effects, such as rotting, decay, discoloration, decoloration or formation of mould.

Microorganisms capable of degrading or altering the industrial materials include, for example, bacteria, fungi,

yeasts, algae and slime organisms. The compositions according to the invention preferably act against fungi, especially moulds, wood-discoloring and wood-destroying fungi (*Ascomycetes, Basidiomycetes, Deuteromycetes* and *Zygomycetes*), and against slime organisms and algae. Examples include microorganisms of the following genera: *Alternaria,* such as *Alternaria tenuis; Aspergillus,* such as *Aspergillus niger; Chaetomium,* such as *Chaetomium globosum; Coniophora,* such as *Coniophora puetana; Lentinus,* such as *Lentinus tigrinus; Penicillium,* such as *Penicillium glaucum; Polyporus,* such as *Polyporus versicolor; Aureobasidium,* such as *Aureobasidium pullulans; Sclerophoma,* such as *Sclerophoma pityophila; Trichoderma,* such as *Trichoderma viride; Ophiostoma* spp., *Ceratocystis* spp., *Humicola* spp., *Petriella* spp., *Trichurus* spp., *Coriolus* spp., *Gloeophyllum* spp., *Pleurotus* spp., *Poria* spp., *Serpula* spp. and *Tyromyces* spp., *Cladosporium* spp., *Paecilomyces* spp. *Mucor* spp., *Escherichia,* such as *Escherichia coli; Pseudomonas,* such as *Pseudomonas aeruginosa; Staphylococcus,* such as *Staphylococcus aureus, Candida* spp. and *Saccharomyces* spp., such as *Saccharomyces cerevisae.*

*Antimycotic Activity*

In addition, the compositions according to the invention also have very good antimycotic activity. They have a very broad antimycotic activity spectrum, especially against dermatophytes and yeasts, moulds and diphasic fungi (for example against *Candida* species, such as *C. albicans, C. glabrata*), and *Epidermophyton floccosum, Aspergillus* species, such as *A. niger* and *A. fumigatus, Trichophyton* species, such as *T. mentagrophytes, Microsporon* species such as *M. canis* and *M. audouinii.* The list of these fungi by no means constitutes a restriction of the mycotic spectrum covered, and is merely of illustrative character.

The compositions according to the invention can therefore be used both in medical and in non-medical applications.

*GMO*

As already mentioned above, it is possible to treat all plants and their parts in accordance with the invention. In a preferred embodiment, wild plant species and plant cultivars, or those obtained by conventional biological breeding methods, such as crossing or protoplast fusion, and also parts thereof, are treated. In a further preferred embodiment, transgenic plants and plant cultivars obtained by genetic engineering methods, if appropriate in combination with conventional methods (Genetically Modified Organisms), and parts thereof are treated. The terms "parts" or "parts of plants" or "plant parts" have been explained above. More preferably, plants of the plant cultivars which are commercially available or are in use are treated in accordance with the invention. Plant cultivars are understood to mean plants which have new properties ("traits") and have been obtained by conventional breeding, by mutagenesis or by recombinant DNA techniques. They can be cultivars, varieties, bio- or genotypes.

The method of treatment according to the invention can be used in the treatment of genetically modified organisms (GMOs), e.g. plants or seeds. Genetically modified plants (or transgenic plants) are plants of which a heterologous gene has been stably integrated into genome. The expression "heterologous gene" essentially means a gene which is provided or assembled outside the plant and when introduced in the nuclear, chloroplastic or mitochondrial genome gives the transformed plant new or improved agronomic or other properties by expressing a protein or polypeptide of interest or by downregulating or silencing other gene(s) which are present in the plant (using for example, antisense technology, cosuppression technology, RNA interference - RNAi - technology or microRNA - miRNA - technology). A heterologous gene that is located in the genome is also called a transgene. A transgene that is defined by its particular location in the plant genome is called a transformation or transgenic event.

Depending on the plant species or plant cultivars, their location and growth conditions (soils, climate, vegetation period, diet), the treatment according to the invention may also result in superadditive ("synergistic") effects. Thus, for example, reduced application rates and/or a widening of the activity spectrum and/or an increase in the activity of the active compounds and compositions which can be used according to the invention, better plant growth, increased tolerance to high or low temperatures, increased tolerance to drought or to water or soil salt content, increased flowering performance, easier harvesting, accelerated maturation, higher harvest yields, bigger fruits, larger plant height, greener leaf color, earlier flowering, higher quality and/or a higher nutritional value of the harvested products, higher sugar concentration within the fruits, better storage stability and/or processability of the harvested products are possible, which exceed the effects which were actually to be expected.

At certain application rates, the compositions according to the invention according to the invention may also have a strengthening effect in plants. Accordingly, they are also suitable for mobilizing the defense system of the plant against attack by unwanted microorganisms. This may, if appropriate, be one of the reasons of the enhanced activity of the compositions according to the invention, for example against fungi. Plant-strengthening (resistance-inducing) substances are to be understood as meaning, in the present context, those substances or combinations of substances which are capable of stimulating the defense system of plants in such a way that, when subsequently inoculated with

unwanted microorganisms, the treated plants display a substantial degree of resistance to these microorganisms. In the present case, harmful microorganisms are to be understood as meaning phytopathogenic fungi, bacteria and viruses. Thus, the substances according to the invention can be employed for protecting plants against attack by the abovementioned pathogens within a certain period of time after the treatment. The period of time within which protection is effected generally extends from 1 to 10 days, preferably 1 to 7 days, after the treatment of the plants with the active compounds.

Plants and plant cultivars which are preferably to be treated according to the invention include all plants which have genetic material which impart particularly advantageous, useful traits to these plants (whether obtained by breeding and/or biotechnological means).

Plants and plant cultivars which are also preferably to be treated according to the invention are resistant against one or more biotic stresses, i.e. said plants show a better defense against animal and microbial pests, such as against nematodes, insects, mites, phytopathogenic fungi, bacteria, viruses and/or viroids.

Examples of nematode or insect resistant plants are described in e.g. U.S. Patent Applications 11/765,491, 11/765,494, 10/926,819, 10/782,020, 12/032,479, 10/783,417, 10/782,096, 11/657,964, 12/192,904, 11/396,808, 12/166,253, 12/166,239, 12/166,124, 12/166,209, 11/762,886, 12/364,335, 11/763,947, 12/252,453, 12/209,354, 12/491,396, 12/497,221, 12/644,632, 12/646,004, 12/701,058, 12/718,059, 12/721,595, 12/638,591.

Plants and plant cultivars which may also be treated according to the invention are those plants which are resistant to one or more abiotic stresses. Abiotic stress conditions may include, for example, drought, cold temperature exposure, heat exposure, osmotic stress, flooding, increased soil salinity, increased mineral exposure, ozone exposure, high light exposure, limited availability of nitrogen nutrients, limited availability of phosphorus nutrients, shade avoidance.

Plants and plant cultivars which may also be treated according to the invention, are those plants characterized by enhanced yield characteristics. Increased yield in said plants can be the result of, for example, improved plant physiology, growth and development, such as water use efficiency, water retention efficiency, improved nitrogen use, enhanced carbon assimilation, improved photosynthesis, increased germination efficiency and accelerated maturation. Yield can furthermore be affected by improved plant architecture (under stress and non-stress conditions), including but not limited to, early flowering, flowering control for hybrid seed production, seedling vigor, plant size, internode number and distance, root growth, seed size, fruit size, pod size, pod or ear number, seed number per pod or ear, seed mass, enhanced seed filling, reduced seed dispersal, reduced pod dehiscence and lodging resistance. Further yield traits include seed composition, such as carbohydrate content, protein content, oil content and composition, nutritional value, reduction in anti-nutritional compounds, improved processability and better storage stability.

Plants that may be treated according to the invention are hybrid plants that already express the characteristic of heterosis or hybrid vigor which results in generally higher yield, vigor, health and resistance towards biotic and abiotic stresses). Such plants are typically made by crossing an inbred male-sterile parent line (the female parent) with another inbred male-fertile parent line (the male parent). Hybrid seed is typically harvested from the male sterile plants and sold to growers. Male sterile plants can sometimes (e.g. in corn) be produced by detasseling, i.e. the mechanical removal of the male reproductive organs (or males flowers) but, more typically, male sterility is the result of genetic determinants in the plant genome. In that case, and especially when seed is the desired product to be harvested from the hybrid plants it is typically useful to ensure that male fertility in the hybrid plants is fully restored. This can be accomplished by ensuring that the male parents have appropriate fertility restorer genes which are capable of restoring the male fertility in hybrid plants that contain the genetic determinants responsible for male-sterility. Genetic determinants for male sterility may be located in the cytoplasm. Examples of cytoplasmic male sterility (CMS) were for instance described in *Brassica* species (WO 92/05251, WO 95/09910, WO 98/27806, WO 05/002324, WO 06/021972 and US 6,229,072). However, genetic determinants for male sterility can also be located in the nuclear genome. Male sterile plants can also be obtained by plant biotechnology methods such as genetic engineering. A particularly useful means of obtaining male-sterile plants is described in WO 89/10396 in which, for example, a ribonuclease such as barnase is selectively expressed in the tapetum cells in the stamens. Fertility can then be restored by expression in the tapetum cells of a ribonuclease inhibitor such as barstar (e.g. WO 91/02069).

Plants or plant cultivars (obtained by plant biotechnology methods such as genetic engineering) which may be treated according to the invention are herbicide-tolerant plants, i.e. plants made tolerant to one or more given herbicides. Such plants can be obtained either by genetic transformation, or by selection of plants containing a mutation imparting such herbicide tolerance.

Herbicide-resistant plants are for example glyphosate-tolerant plants, i.e. plants made tolerant to the herbicide glyphosate or salts thereof. Plants can be made tolerant to glyphosate through different means. For example, glyphosate-tolerant plants can be obtained by transforming the plant with a gene encoding the enzyme 5-enolpyruvylshikimate-3-phosphate synthase (EPSPS). Examples of such EPSPS genes are the AroA gene (mutant CT7) of the bacterium *Salmonella typhimurium* (Science 1983, 221, 370-371), the CP4 gene of the bacterium Ag*robacterium sp.* (Curr. Tropics Plant Physiol. 1992, 7, 139-145), the genes encoding a Petunia EPSPS (Science 1986, 233, 478-481), a Tomato EPSPS (J. Biol. Chem. 1988, 263, 4280-4289), or an Eleusine EPSPS (WO 01/66704). It can also be a mutated EPSPS as described in for example EP 0837944, WO 00/66746, WO 00/66747 or WO 02/26995. Glyphosate-tolerant plants can

also be obtained by expressing a gene that encodes a glyphosate oxido-reductase enzyme as described in US 5,776,760 and US 5,463,175. Glyphosate-tolerant plants can also be obtained by expressing a gene that encodes a glyphosate acetyl transferase enzyme as described in for example WO 02/036782, WO 03/092360, WO 2005/012515 and WO 2007/024782. Glyphosate-tolerant plants can also be obtained by selecting plants containing naturally-occurring mutations of the above-mentioned genes, as described in for example WO 01/024615 or WO 03/013226. Plants expressing EPSPS genes that confer glyphosate tolerance are described in e.g. U.S. Patent Applications 11/517,991, 10/739,610, 12/139,408, 12/352,532, 11/312,866, 11/315,678, 12/421,292, 11/400,598, 11/651,752, 11/681,285, 11/605,824, 12/468,205, 11/760,570, 11/762,526, 11/769,327, 11/769,255, 11/943801 or 12/362,774. Plants comprising other genes that confer glyphosate tolerance, such as decarboxylase genes, are described in e.g. U.S. Patent Applications 11/588,811, 11/185,342, 12/364,724, 11/185,560 or 12/423,926.

Other herbicide resistant plants are for example plants that are made tolerant to herbicides inhibiting the enzyme glutamine synthase, such as bialaphos, phosphinothricin or glufosinate. Such plants can be obtained by expressing an enzyme detoxifying the herbicide or a mutant glutamine synthase enzyme that is resistant to inhibition, e.g. described in U.S. Patent Application 11/760,602. One such efficient detoxifying enzyme is an enzyme encoding a phosphinothricin acetyltransferase (such as the bar or pat protein from *Streptomyces* species). Plants expressing an exogenous phosphinothricin acetyltransferase are for example described in U.S. Patents 5,561,236; 5,648,477; 5,646,024; 5,273,894; 5,637,489; 5,276,268; 5,739,082; 5,908,810 and 7,112,665.

Further herbicide-tolerant plants are also plants that are made tolerant to the herbicides inhibiting the enzyme hydroxyphenylpyruvatedioxygenase (HPPD). HPPD is an enzyme that catalyze the reaction in which parahydroxyphenylpyruvate (HPP) is transformed into homogentisate. Plants tolerant to HPPD-inhibitors can be transformed with a gene encoding a naturally-occurring resistant HPPD enzyme, or a gene encoding a mutated or chimeric HPPD enzyme as described in WO 96/38567, WO 99/24585, WO 99/24586, WO 09/144079, WO 02/046387, or US 6,768,044. Tolerance to HPPD-inhibitors can also be obtained by transforming plants with genes encoding certain enzymes enabling the formation of homogentisate despite the inhibition of the native HPPD enzyme by the HPPD-inhibitor. Such plants and genes are described in WO 99/34008 and WO 02/36787. Tolerance of plants to HPPD inhibitors can also be improved by transforming plants with a gene encoding an enzyme having prephenate deshydrogenase (PDH) activity in addition to a gene encoding an HPPD-tolerant enzyme, as described in WO 04/024928. Further, plants can be made more tolerant to HPPD-inhibitor herbicides by adding into their genome a gene encoding an enzyme capable of metabolizing or degrading HPPD inhibitors, such as the CYP450 enzymes shown in WO 2007/103567 and WO 2008/150473.

Still further herbicide resistant plants are plants that are made tolerant to acetolactate synthase (ALS) inhibitors. Known ALS-inhibitors include, for example, sulfonylurea, imidazolinone, triazolopyrimidines, pryimidinyoxy-(thio)benzoates, and/or sulfonylaminocarbonyltriazolinone herbicides. Different mutations in the ALS enzyme (also known as acetohydroxyacid synthase, AHAS) are known to confer tolerance to different herbicides and groups of herbicides, as described for example in Tranel and Wright (Weed Science 2002, 50, 700-712), but also, in U.S. Patents 5,605,011, 5,378,824, 5,141,870, and 5,013,659. The production of sulfonylurea-tolerant plants and imidazolinone-tolerant plants is described in U.S. Patents 5,605,011; 5,013,659; 5,141,870; 5,767,361; 5,731,180; 5,304,732; 4,761,373; 5,331,107; 5,928,937; and 5,378,824; and WO 96/33270. Other imidazolinone-tolerant plants are also described in for example WO 2004/040012, WO 2004/106529, WO 2005/020673, WO 2005/093093, WO 2006/007373, WO 2006/015376, WO 2006/024351, and WO 2006/060634. Further sulfonylurea- and imidazolinone-tolerant plants are also described in for example WO 2007/024782 and U.S. Patent Application 61/288958.

Other plants tolerant to imidazolinone and/or sulfonylurea can be obtained by induced mutagenesis, selection in cell cultures in the presence of the herbicide or mutation breeding as described for example for soybeans in US 5,084,082, for rice in WO 97/41218, for sugar beet in US 5,773,702 and WO 99/057965, for lettuce in US 5,198,599, or for sunflower in WO 01/065922.

Plants or plant cultivars (obtained by plant biotechnology methods such as genetic engineering) which may also be treated according to the invention are insect-resistant transgenic plants, i.e. plants made resistant to attack by certain target insects. Such plants can be obtained by genetic transformation, or by selection of plants containing a mutation imparting such insect resistance.

An "insect-resistant transgenic plant", as used herein, includes any plant containing at least one transgene comprising a coding sequence encoding:

1) an insecticidal crystal protein from *Bacillus thuringiensis* or an insecticidal portion thereof, such as the insecticidal crystal proteins listed by Crickmore et al. (1998, Microbiology and Molecular Biology Reviews, 62: 807-813), updated by Crickmore et al. (2005) at the *Bacillus thuringiensis* toxin nomenclature, online at: http://www.lifesci.sussex.ac.uk/Home/Neil Crickmore/Bt/), or insecticidal portions thereof, e.g., proteins of the Cry protein classes Cry1Ab, Cry1Ac, Cry1B, Cry1C, Cry1D, Cry1F, Cry2Ab, Cry3Aa, or Cry3Bb or insecticidal portions thereof (e.g. EP-A 1 999 141 and WO 2007/107302), or such proteins encoded by synthetic genes as e.g. described in and U.S. Patent Application 12/249,016 ; or

2) a crystal protein from *Bacillus thuringiensis* or a portion thereof which is insecticidal in the presence of a second other crystal protein from *Bacillus thuringiensis* or a portion thereof, such as the binary toxin made up of the Cry34 and Cry35 crystal proteins (Nat. Biotechnol. 2001, 19, 668-72; Applied Environm. Microbiol. 2006, 71, 1765-1774) or the binary toxin made up of the Cry1A or Cry1F proteins and the Cry2Aa or Cry2Ab or Cry2Ae proteins (U.S. Patent Application 12/214,022 and EP-A 2 300 618); or

3) a hybrid insecticidal protein comprising parts of different insecticidal crystal proteins from *Bacillus thuringiensis,* such as a hybrid of the proteins of 1) above or a hybrid of the proteins of 2) above, e.g., the Cry1A. 105 protein produced by corn event MON89034 (WO 2007/027777); or

4) a protein of any one of 1) to 3) above wherein some, particularly 1 to 10, amino acids have been replaced by another amino acid to obtain a higher insecticidal activity to a target insect species, and/or to expand the range of target insect species affected, and/or because of changes introduced into the encoding DNA during cloning or transformation, such as the Cry3Bb1 protein in corn events MON863 or MON88017, or the Cry3A protein in corn event MIR604; or

5) an insecticidal secreted protein from *Bacillus thuringiensis* or *Bacillus cereus,* or an insecticidal portion thereof, such as the vegetative insecticidal (VIP) proteins listed at: http://www.lifesci.sussex.ac.uk/home/Neil_Crickmore/Bt/vip.html, e.g., proteins from the VIP3Aa protein class; or

6) a secreted protein from *Bacillus thuringiensis* or *Bacillus cereus* which is insecticidal in the presence of a second secreted protein from *Bacillus thuringiensis* or *B. cereus,* such as the binary toxin made up of the VIP1A and VIP2A proteins (WO 94/21795); or

7) a hybrid insecticidal protein comprising parts from different secreted proteins from *Bacillus thuringiensis* or *Bacillus cereus,* such as a hybrid of the proteins in 1) above or a hybrid of the proteins in 2) above; or

8) a protein of any one of 5) to 7) above wherein some, particularly 1 to 10, amino acids have been replaced by another amino acid to obtain a higher insecticidal activity to a target insect species, and/or to expand the range of target insect species affected, and/or because of changes introduced into the encoding DNA during cloning or transformation (while still encoding an insecticidal protein), such as the VIP3Aa protein in cotton event COT 102; or

9) a secreted protein from *Bacillus thuringiensis* or *Bacillus cereus* which is insecticidal in the presence of a crystal protein from *Bacillus thuringiensis,* such as the binary toxin made up of VIP3 and Cry1A or Cry1F (U.S. Patent Applications 61/126083 and 61/195019), or the binary toxin made up of the VIP3 protein and the Cry2Aa or Cry2Ab or Cry2Ae proteins (U.S. Patent Application 12/214,022 and EP-A 2300618).

10) a protein of 9) above wherein some, particularly 1 to 10, amino acids have been replaced by another amino acid to obtain a higher insecticidal activity to a target insect species, and/or to expand the range of target insect species affected, and/or because of changes introduced into the encoding DNA during cloning or transformation (while still encoding an insecticidal protein)

Of course, an insect-resistant transgenic plant, as used herein, also includes any plant comprising a combination of genes encoding the proteins of any one of the above classes 1 to 10. In one embodiment, an insect-resistant plant contains more than one transgene encoding a protein of any one of the above classes 1 to 10, to expand the range of target insect species affected when using different proteins directed at different target insect species, or to delay insect resistance development to the plants by using different proteins insecticidal to the same target insect species but having a different mode of action, such as binding to different receptor binding sites in the insect.

An "insect-resistant transgenic plant", as used herein, further includes any plant containing at least one transgene comprising a sequence producing upon expression a double-stranded RNA which upon ingestion by a plant insect pest inhibits the growth of this insect pest, as described e.g. in WO 2007/080126, WO 2006/129204, WO 2007/074405, WO 2007/080127 and WO 2007/035650.

Plants or plant cultivars (obtained by plant biotechnology methods such as genetic engineering) which may also be treated according to the invention are tolerant to abiotic stresses. Such plants can be obtained by genetic transformation, or by selection of plants containing a mutation imparting such stress resistance. Particularly useful stress tolerance plants include:

1) plants which contain a transgene capable of reducing the expression and/or the activity of poly(ADP-ribose)

polymerase (PARP) gene in the plant cells or plants as described in WO 00/04173, WO 2006/045633, EP-A 1 807 519, or EP-A 2 018 431.

2) plants which contain a stress tolerance enhancing transgene capable of reducing the expression and/or the activity of the PARG encoding genes of the plants or plants cells, as described e.g. in WO 2004/090140.

3) plants which contain a stress tolerance enhancing transgene coding for a plant-functional enzyme of the nicotineamide adenine dinucleotide salvage synthesis pathway including nicotinamidase, nicotinate phosphoribosyltransferase, nicotinic acid mononucleotide adenyl transferase, nicotinamide adenine dinucleotide synthetase or nicotine amide phosphorybosyltransferase as described e.g. in EP-A 1 794 306, WO 2006/133827, WO 2007/107326, EP-A 1 999 263, or WO 2007/107326.

Plants or plant cultivars (obtained by plant biotechnology methods such as genetic engineering) which may also be treated according to the invention show altered quantity, quality and/or storage-stability of the harvested product and/or altered properties of specific ingredients of the harvested product such as:

1) transgenic plants which synthesize a modified starch, which in its physical-chemical characteristics, in particular the amylose content or the amylose/amylopectin ratio, the degree of branching, the average chain length, the side chain distribution, the viscosity behaviour, the gelling strength, the starch grain size and/or the starch grain morphology, is changed in comparison with the synthesised starch in wild type plant cells or plants, so that this is better suited for special applications. Said transgenic plants synthesizing a modified starch are disclosed, for example, in EP-A 0 571427, WO 95/04826, EP-A 0 719 338, WO 96/15248, WO 96/19581, WO 96/27674, WO 97/11188, WO 97/26362, WO 97/32985, WO 97/42328, WO 97/44472, WO 97/45545, WO 98/27212, WO 98/40503, WO 99/58688, WO 99/58690, WO 99/58654, WO 00/08184, WO 00/08185, WO 00/08175, WO 00/28052, WO 00/77229, WO 01/12782, WO 01/12826, WO 02/101059, WO 03/071860, WO 04/056999, WO 05/030942, WO 2005/030941, WO 2005/095632, WO 2005/095617, WO 2005/095619, WO 2005/095618, WO 2005/123927, WO 2006/018319, WO 2006/103107, WO 2006/108702, WO 2007/009823, WO 00/22140, WO 2006/063862, WO 2006/072603, WO 02/034923, WO 2008/017518, WO 2008/080630, WO 2008/080631, EP 07090007.1, WO 2008/090008, WO 01/14569, WO 02/79410, WO 03/33540, WO 2004/078983, WO 01/19975, WO 95/26407, WO 96/34968, WO 98/20145, WO 99/12950, WO 99/66050, WO 99/53072, US 6,734,341, WO 00/11192, WO 98/22604, WO 98/32326, WO 01/98509, WO 01/98509, WO 2005/002359, US 5,824,790, US 6,013,861, WO 94/04693, WO 94/09144, WO 94/11520, WO 95/35026, WO 97/20936, WO 2010/012796, WO 2010/003701,

2) transgenic plants which synthesize non starch carbohydrate polymers or which synthesize non starch carbohydrate polymers with altered properties in comparison to wild type plants without genetic modification. Examples are plants producing polyfi-uctose, especially of the inulin and levan-type, as disclosed in EP-A 0 663 956, WO 96/01904, WO 96/21023, WO 98/39460, and WO 99/24593, plants producing alpha-1,4-glucans as disclosed in WO 95/31553, US 2002031826, US 6,284,479, US 5,712,107, WO 97/47806, WO 97/47807, WO 97/47808 and WO 00/14249, plants producing alpha-1,6 branched alpha-1,4-glucans, as disclosed in WO 00/73422, plants producing alternan, as disclosed in e.g. WO 00/47727, WO 00/73422, EP 06077301.7, US 5,908,975 and EP-A 0 728 213,

3) transgenic plants which produce hyaluronan, as for example disclosed in WO 2006/032538, WO 2007/039314, WO 2007/039315, WO 2007/039316, JP-A 2006-304779, and WO 2005/012529.

4) transgenic plants or hybrid plants, such as onions with characteristics such as 'high soluble solids content', 'low pungency' (LP) and/or 'long storage' (LS), as described in U.S. Patent Applications 12/020,360 and 61/054,026.

Plants or plant cultivars (that can be obtained by plant biotechnology methods such as genetic engineering) which may also be treated according to the invention are plants, such as cotton plants, with altered fiber characteristics. Such plants can be obtained by genetic transformation, or by selection of plants contain a mutation imparting such altered fiber characteristics and include:

a) Plants, such as cotton plants, containing an altered form of cellulose synthase genes as described in WO 98/00549.

b) Plants, such as cotton plants, containing an altered form of rsw2 or rsw3 homologous nucleic acids as described in WO 2004/053219.

c) Plants, such as cotton plants, with increased expression of sucrose phosphate synthase as described in WO

01/17333.

d) Plants, such as cotton plants, with increased expression of sucrose synthase as described in WO 02/45485.

e) Plants, such as cotton plants, wherein the timing of the plasmodesmatal gating at the basis of the fiber cell is altered, e.g. through downregulation of fiber-selective β-1,3-glucanase as described in WO 2005/017157, or as described in WO 2009/143995.

f) Plants, such as cotton plants, having fibers with altered reactivity, e.g. through the expression of N-acetylglucosaminetransferase gene including nodC and chitin synthase genes as described in WO 2006/136351.

Plants or plant cultivars (that can be obtained by plant biotechnology methods such as genetic engineering) which may also be treated according to the invention are plants, such as oilseed rape or related Brassica plants, with altered oil profile characteristics. Such plants can be obtained by genetic transformation, or by selection of plants contain a mutation imparting such altered oil profile characteristics and include:

a) Plants, such as oilseed rape plants, producing oil having a high oleic acid content as described e.g. in US 5,969,169, US 5,840,946 or US 6,323,392 or US 6,063,947

b) Plants such as oilseed rape plants, producing oil having a low linolenic acid content as described in US 6,270,828, US 6,169,190, or US 5,965,755

c) Plant such as oilseed rape plants, producing oil having a low level of saturated fatty acids as described e.g. in US 5,434,283 or U.S. Patent Application 12/668303

Plants or plant cultivars (that can be obtained by plant biotechnology methods such as genetic engineering) which may also be treated according to the invention are plants, such as oilseed rape or related Brassica plants, with altered seed shattering characteristics. Such plants can be obtained by genetic transformation, or by selection of plants contain a mutation imparting such altered seed shattering characteristics and include plants such as oilseed rape plants with delayed or reduced seed shattering as described in U.S. Patent Application 61/135,230, WO 2009/068313 and WO 2010/006732.

Plants or plant cultivars (that can be obtained by plant biotechnology methods such as genetic engineering) which may also be treated according to the invention are plants, such as Tobacco plants, with altered post-translational protein modification patterns, for example as described in WO 2010/121818 and WO 2010/145846.

Particularly useful transgenic plants which may be treated according to the invention are plants containing transformation events, or combination of transformation events, that are the subject of petitions for non-regulated status, in the United States of America, to the Animal and Plant Health Inspection Service (APHIS) of the United States Department of Agriculture (USDA) whether such petitions are granted or are still pending. At any time this information is readily available from APHIS (4700 River Road, Riverdale, MD 20737, USA), for instance on its internet site (URL http://www.aphis.usda.gov/brs/not_reg.html). On the filing date of this application the petitions for nonregulated status that were pending with APHIS or granted by APHIS were those which contains the following information:

- Petition: the identification number of the petition. Technical descriptions of the transformation events can be found in the individual petition documents which are obtainable from APHIS, for example on the APHIS website, by reference to this petition number. These descriptions are herein incorporated by reference.

- Extension of Petition: reference to a previous petition for which an extension is requested.

- Institution: the name of the entity submitting the petition.

- Regulated article: the plant species concerned.

- Transgenic phenotype: the trait conferred to the plants by the transformation event.

- Transformation event or line: the name of the event or events (sometimes also designated as lines or lines) for which nonregulated status is requested.

- APHIS documents: various documents published by APHIS in relation to the Petition and which can be requested

with APHIS.

Additional particularly useful plants containing single transformation events or combinations of transformation events are listed for example in the databases from various national or regional regulatory agencies (see for example http://gmoinfo.jrc.it/gmp_browse.aspx and http://www.agbios.com/dbase.php).

Particularly useful transgenic plants which may be treated according to the invention are plants containing transformation events, or a combination of transformation events, and that are listed for example in the databases for various national or regional regulatory agencies including Event 531/ PV-GHBK04 (cotton, insect control, described in WO 2002/040677), Event 1143-14A (cotton, insect control, not deposited, described in WO 06/128569); Event 1143-51B (cotton, insect control, not deposited, described in WO 06/128570); Event 1445 (cotton, herbicide tolerance, not deposited, described in US-A 2002-120964 or WO 02/034946Event 17053 (rice, herbicide tolerance, deposited as PTA-9843, described in WO 10/117737); Event 17314 (rice, herbicide tolerance, deposited as PTA-9844, described in WO 10/117735); Event 281-24-236 (cotton, insect control - herbicide tolerance, deposited as PTA-6233, described in WO 05/103266 or US-A 2005-216969); Event 3006-210-23 (cotton, insect control - herbicide tolerance, deposited as PTA-6233, described in US-A 2007-143876 or WO 05/103266); Event 3272 (corn, quality trait, deposited as PTA-9972, described in WO 06/098952 or US-A 2006-230473); Event 33391 (wheat, herbicide tolerance, deposited as PTA-2347, described in WO 2002/027004), Event 40416 (corn, insect control - herbicide tolerance, deposited as ATCC PTA-11508, described in WO 11/075593); Event 43A47 (corn, insect control - herbicide tolerance, deposited as ATCC PTA-11509, described in WO 11/075595); Event 5307 (corn, insect control, deposited as ATCC PTA-9561, described in WO 10/077816); Event ASR-368 (bent grass, herbicide tolerance, deposited as ATCC PTA-4816, described in US-A 2006-162007 or WO 04/053062); Event B16 (corn, herbicide tolerance, not deposited, described in US-A 2003-126634); Event BPS-CV127-9 (soybean, herbicide tolerance, deposited as NCIMB No. 41603, described in WO 10/080829); Event BLR1 (oilseed rape, restoration of male sterility, deposited as NCIMB 41193, described in WO 2005/074671), Event CE43-67B (cotton, insect control, deposited as DSM ACC2724, described in US-A 2009-217423 or WO 06/128573); Event CE44-69D (cotton, insect control, not deposited, described in US-A 2010-0024077); Event CE44-69D (cotton, insect control, not deposited, described in WO 06/128571); Event CE46-02A (cotton, insect control, not deposited, described in WO 06/128572); Event COT102 (cotton, insect control, not deposited, described in US-A 2006-130175 or WO 04/039986); Event COT202 (cotton, insect control, not deposited, described in US-A 2007-067868 or WO 05/054479); Event COT203 (cotton, insect control, not deposited, described in WO 05/054480););); Event DAS21606-3 / 1606 (soybean, herbicide tolerance, deposited as PTA-11028, described in WO 012/033794), Event DAS40278 (corn, herbicide tolerance, deposited as ATCC PTA-10244, described in WO 11/022469); Event DAS-44406-6 / pDAB8264.44.06.1 (soybean, herbicide tolerance, deposited as PTA-11336, described in WO 2012/075426), Event DAS-14536-7 /pDAB8291.45.36.2 (soybean, herbicide tolerance, deposited as PTA-11335, described in WO 2012/075429), Event DAS-59122-7 (corn, insect control - herbicide tolerance, deposited as ATCC PTA 11384 , described in US-A 2006-070139); Event DAS-59132 (corn, insect control - herbicide tolerance, not deposited, described in WO 09/100188); Event DAS68416 (soybean, herbicide tolerance, deposited as ATCC PTA-10442, described in WO 11/066384 or WO 11/066360); Event DP-098140-6 (corn, herbicide tolerance, deposited as ATCC PTA-8296, described in US-A 2009-137395 or WO 08/112019); Event DP-305423-1 (soybean, quality trait, not deposited, described in US-A 2008-312082 or WO 08/054747); Event DP-32138-1 (corn, hybridization system, deposited as ATCC PTA-9158, described in US-A 2009-0210970 or WO 09/103049); Event DP-356043-5 (soybean, herbicide tolerance, deposited as ATCC PTA-8287, described in US-A 2010-0184079 or WO 08/002872); Event EE-1 (brinjal, insect control, not deposited, described in WO 07/091277); Event FI117 (corn, herbicide tolerance, deposited as ATCC 209031, described in US-A 2006-059581 or WO 98/044140); Event FG72 (soybean, herbicide tolerance, deposited as PTA-11041, described in WO 2011/063413), Event GA21 (corn, herbicide tolerance, deposited as ATCC 209033, described in US-A 2005-086719 or WO 98/044140); Event GG25 (corn, herbicide tolerance, deposited as ATCC 209032, described in US-A 2005-188434 or WO 98/044140); Event GHB119 (cotton, insect control - herbicide tolerance, deposited as ATCC PTA-8398, described in WO 08/151780); Event GHB614 (cotton, herbicide tolerance, deposited as ATCC PTA-6878, described in US-A 2010-050282 or WO 07/017186); Event GJ11 (corn, herbicide tolerance, deposited as ATCC 209030, described in US-A 2005-188434 or WO 98/044140); Event GM RZ13 (sugar beet, virus resistance , deposited as NCIMB-41601, described in WO 10/076212); Event H7-1 (sugar beet, herbicide tolerance, deposited as NCIMB 41158 or NCIMB 41159, described in US-A 2004-172669 or WO 04/074492); Event JOPLIN1 (wheat, disease tolerance, not deposited, described in US-A 2008-064032); Event LL27 (soybean, herbicide tolerance, deposited as NCIMB41658, described in WO 06/108674 or US-A 2008-320616); Event LL55 (soybean, herbicide tolerance, deposited as NCIMB 41660, described in WO 06/108675 or US-A 2008-196127); Event LLcotton25 (cotton, herbicide tolerance, deposited as ATCC PTA-3343, described in WO 03/013224 or US-A 2003-097687); Event LLRICE06 (rice, herbicide tolerance, deposited as ATCC 203353, described in US 6,468,747 or WO 00/026345); Event LLRice62 (rice, herbicide tolerance, deposited as ATCC 203352, described in WO 2000/026345), Event LLRICE601 (rice, herbicide tolerance, deposited as ATCC PTA-2600, described in US-A 2008-2289060 or WO 00/026356); Event LY038 (corn, quality trait, deposited as ATCC PTA-5623, described

in US-A 2007-028322 or WO 05/061720); Event MIR162 (corn, insect control, deposited as PTA-8166, described in US-A 2009-300784 or WO 07/142840); Event MIR604 (corn, insect control, not deposited, described in US-A 2008-167456 or WO 05/103301); Event MON15985 (cotton, insect control, deposited as ATCC PTA-2516, described in US-A 2004-250317 or WO 02/100163); Event MON810 (corn, insect control, not deposited, described in US-A 2002-102582); Event MON863 (corn, insect control, deposited as ATCC PTA-2605, described in WO 04/011601 or US-A 2006-095986); Event MON87427 (corn, pollination control, deposited as ATCC PTA-7899, described in WO 11/062904); Event MON87460 (corn, stress tolerance, deposited as ATCC PTA-8910, described in WO 09/111263 or US-A 2011-0138504); Event MON87701 (soybean, insect control, deposited as ATCC PTA-8194, described in US-A 2009-130071 or WO 09/064652); Event MON87705 (soybean, quality trait - herbicide tolerance, deposited as ATCC PTA-9241, described in US-A 2010-0080887 or WO 10/037016); Event MON87708 (soybean, herbicide tolerance, deposited as ATCC PTA-9670, described in WO 11/034704); Event MON87712 (soybean, yield, deposited as PTA-10296, described in WO 2012/051199), Event MON87754 (soybean, quality trait, deposited as ATCC PTA-9385, described in WO 10/024976); Event MON87769 (soybean, quality trait, deposited as ATCC PTA-8911, described in US-A 2011-0067141 or WO 09/102873); Event MON88017 (corn, insect control - herbicide tolerance, deposited as ATCC PTA-5582, described in US-A 2008-028482 or WO 05/059103); Event MON88913 (cotton, herbicide tolerance, deposited as ATCC PTA-4854, described in WO 04/072235 or US-A 2006-059590); Event MON88302 (oilseed rape, herbicide tolerance, deposited as PTA-10955, described in WO 2011/153186), Event MON88701 (cotton, herbicide tolerance, deposited as PTA-11754, described in WO 2012/134808), Event MON89034 (corn, insect control, deposited as ATCC PTA-7455, described in WO 07/140256 or US-A 2008-260932); Event MON89788 (soybean, herbicide tolerance, deposited as ATCC PTA-6708, described in US-A 2006-282915 or WO 06/130436); Event MS11 (oilseed rape, pollination control - herbicide tolerance, deposited as ATCC PTA-850 or PTA-2485, described in WO 01/031042); Event MS8 (oilseed rape, pollination control - herbicide tolerance, deposited as ATCC PTA-730, described in WO 01/041558 or US-A 2003-188347); Event NK603 (corn, herbicide tolerance, deposited as ATCC PTA-2478, described in US-A 2007-292854); Event PE-7 (rice, insect control, not deposited, described in WO 08/114282); Event RF3 (oilseed rape, pollination control - herbicide tolerance, deposited as ATCC PTA-730, described in WO 01/041558 or US-A 2003-188347); Event RT73 (oilseed rape, herbicide tolerance, not deposited, described in WO 02/036831 or US-A 2008-070260); Event SYHT0H2 / SYN-000H2-5 (soybean, herbicide tolerance, deposited as PTA-11226, described in WO 2012/082548), Event T227-1 (sugar beet, herbicide tolerance, not deposited, described in WO 02/44407 or US-A 2009-265817); Event T25 (corn, herbicide tolerance, not deposited, described in US-A 2001-029014 or WO 01/051654); Event T304-40 (cotton, insect control - herbicide tolerance, deposited as ATCC PTA-8171, described in US-A 2010-077501 or WO 08/122406); Event T342-142 (cotton, insect control, not deposited, described in WO 06/128568); Event TC1507 (corn, insect control - herbicide tolerance, not deposited, described in US-A 2005-039226 or WO 04/099447); Event VIP1034 (corn, insect control - herbicide tolerance, deposited as ATCC PTA-3925., described in WO 03/052073), Event 32316 (corn, insect control-herbicide tolerance, deposited as PTA-11507, described in WO 11/084632), Event 4114 (corn, insect control-herbicide tolerance, deposited as PTA-11506, described in WO 11/084621), event EE-GM3 / FG72 (soybean, herbicide tolerance, ATCC Accession N° PTA-11041, WO 2011/063413A2), event DAS-68416-4 (soybean, herbicide tolerance, ATCC Accession N° PTA-10442, WO2 011/066360A1), event DAS-68416-4 (soybean, herbicide tolerance, ATCC Accession N° PTA-10442, WO 2011/066384A1), event DP-040416-8 (corn, insect control, ATCC Accession N° PTA-11508, WO 2011/075593A1), event DP-043A47-3 (corn, insect control, ATCC Accession N° PTA-11509, WO 2011/075595A1), event DP-004114-3 (corn, insect control, ATCC Accession N° PTA-11506, WO 2011/084621A1), event DP-032316-8 (corn, insect control, ATCC Accession N° PTA-11507, WO 2011/084632A1), event MON-88302-9 (oilseed rape, herbicide tolerance, ATCC Accession N° PTA-10955, WO 2011/153186A1), event DAS-21606-3 (soybean, herbicide tolerance, ATCC Accession No. PTA-11028, WO 2012/033794A2), event MON-87712-4 (soybean, quality trait, ATCC Accession N°. PTA-10296, WO 2012/051199A2), event DAS-44406-6 (soybean, stacked herbicide tolerance, ATCC Accession N°. PTA-11336, WO 2012/075426A1), event DAS-14536-7 (soybean, stacked herbicide tolerance, ATCC Accession N°. PTA-11335, WO 2012/075429A1), event SYN-000H2-5 (soybean, herbicide tolerance, ATCC Accession N°. PTA-11226, WO 2012/082548A2), event DP-061061-7 (oilseed rape, herbicide tolerance, no deposit N° available, WO 2012071039A1), event DP-073496-4 (oilseed rape, herbicide tolerance, no deposit N° available, US2012131692), event 8264.44.06.1 (soybean, stacked herbicide tolerance, Accession N° PTA-11336, WO 2012075426A2), event 8291.45.36.2 (soybean, stacked herbicide tolerance, Accession N°. PTA-11335, WO 2012075429A2), event SYHT0H2 (soybean, ATCC Accession N°. PTA-11226, WO 2012/082548A2), event MON88701 (cotton, ATCC Accession N° PTA-11754, WO 2012/134808A1), event KK179-2 (alfalfa, ATCC Accession N° PTA-11833, WO2013003558A1), event pDAB8264.42.32.1 (soybean, stacked herbicide tolerance, ATCC Accession N° PTA-11993, WO 2013010094A1), event MZDT09Y (corn, ATCC Accession N° PTA-13025, WO 2013012775A1), event KK179-2 (alfalfa, ATCC Accession N° PTA-11833, WO2013003558A1), event pDAB8264.42.32.1 (soybean, stacked herbicide tolerance, ATCC Accession N° PTA-1 1993, WO2013010094A1), event MZDT09Y (corn, ATCC Accession N° PTA- 13025, WO2013012775A1), event VCO-01981-5 (corn, herbicide tolerance, NCIMB Accession N° 41842, WO2013014241A1), event DAS-81419-2 X DAS-68416-4 (soybean stacked insect resistance and herbicide tolerance, ATCC Accession N° PTA- 10442,

WO2013016516A1), event DAS-81419-2 (soybean stacked insect resistance and herbicide tolerance, ATCC Accession N° PTA-12006, WO2013016527A1), event HCEM485 (corn, herbicide tolerance, ATCC Accession N° PTA-12014, WO2013025400A1), event pDAB4468.18.07.1 (cotton, herbicide tolerance, ATCC Accession N° PTA-12456, WO2013112525A2), event pDAB4468.19.10.3 (cotton, herbicide tolerance, ATCC Accession N° PTA-12457, WO2013112527A1). *Application Rates and*

*Timing*

When using the compositions according to the invention as fungicides, the application rates can be varied within a relatively wide range, depending on the kind of application. The application rate of the compositions according to the invention is

- in the case of treatment of plant parts, for example leaves: from 0.1 to 10 000 g/ha, preferably from 10 to 1000 g/ha, more preferably from 10 to 800 g/ha, even more preferably from 50 to 300 g/ha (in the case of application by watering or dripping, it is even possible to reduce the application rate, especially when inert substrates such as rockwool or perlite are used);
- in the case of seed treatment: from 2 to 200 g per 100 kg of seed, preferably from 3 to 150 g per 100 kg of seed, more preferably from 2.5 to 25 g per 100 kg of seed, even more preferably from 2.5 to 12.5 g per 100 kg of seed;
- in the case of soil treatment: from 0.1 to 10 000 g/ha, preferably from 1 to 5000 g/ha.

These application rates are merely by way of example and are not limiting for the purposes of the invention.

The compositions according to the invention can thus be used to protect plants from attack by the pathogens mentioned for a certain period of time after treatment. The period for which protection is provided extends generally for 1 to 28 days, preferably for 1 to 14 days, more preferably for 1 to 10 days, most preferably for 1 to 7 days, after the treatment of the plants with the active ingredients, or for up to 200 days after a seed treatment.

The method of treatment according to the invention also provides the use or application of compounds (A) and (B) and/or (C) in a simultaneous, separate or sequential manner. If the single active ingredients are applied in a sequential manner, i.e. at different times, they are applied one after the other within a reasonably short period, such as a few hours or days. Preferably the order of applying the compounds (A) and (B) and/or (C) is not essential for working the present invention.

The plants listed can particularly advantageously be treated in accordance with the invention with the compounds of the general formula (I) and the inventive compositions. The preferred ranges stated above for the active ingredients or compositions also apply to the treatment of these plants. Particular emphasis is given to the treatment of plants with the compounds or compositions specifically mentioned in the present text.

The invention is illustrated by the examples below. However, the invention is not limited to the examples.

Preparation example 1 : Preparation of 3-(difluoromethyl)-5-fluoro-N-methoxy-1-methyl-N-[1-(2,4,6-trichlorophenyl)propan-2-yl]-1H-pyrazole-4-carboxamide (compound (I-2))

In a 13 mL Chemspeed™ vial are distributed 3 mL of a 0.2 M solution of N-methoxy-1-(2,4,6-trichlorophenyl)propan-2-amine (0.60 mmol) in dichloromethane followed by 100 uL of triethylamine. 3 mL of a 0.22 M solution of 3-(difluoromethyl)-5-fluoro-1-methyl-1H-pyrazole-4-carbonyl chloride (0.66 mmol) in dichloromethane are added and the mixture is then stirred at room temperature overnight. The mixture is poured over a dual cartridge silica (2 g) + basic alumina (2 g) and eluted with 3 x 6 mL of acetonitrile. The solvents are removed and the crude amide is purified by preparative HPLC-MS to provide 121 mg (45% yield) of 3-(difluoromethyl)-5-fluoro-N-methoxy-1-methyl-N-[1-(2,4,6-trichlorophenyl)propan-2-yl]-1H-pyrazole-4-carboxamide (M+H = 444). logP[a] = 4.29.

[a] Measurement of LC-MS was done at pH 2,7 with 0,1 % formic acid in water and with acetonitrile (contains 0,1% formic acid) as eluent with a linear gradient from 10 % acetonitrile to 95 % acetonitrile.

Measurement of logP values was performed according EEC directive 79/831 Annex V.A8 by HPLC (High Performance Liquid Chromatography) on reversed phase columns with the following methods:

Calibration was done with not branched alkan2-ones (with 3 to 16 carbon atoms) with known logP-values (measurement of logP values using retention times with linear interpolation between successive alkanones). lambda-maX-values were determined using UV-spectra from 200 nm to 400 nm and the peak values of the chromatographic signals.

**Biological Examples**

The advanced fungicidal activity of the active compound combinations according to the invention is evident

from the example below. While the individual active compounds exhibit weaknesses with regard to the fungicidal activity, the combinations have an activity which exceeds a simple addition of activities.

A synergistic effect of fungicides is always present when the fungicidal activity of the active compound combinations exceeds the total of the activities of the active compounds when applied individually. The expected activity for a given combination of two active compounds can be calculated as follows (cf. Col-by, S.R., "Calculating Synergistic and Antagonistic Responses of Herbicide Combinations", Weeds 1967, 15, 20-22):

If

X is the efficacy when active compound A is applied at an application rate of m ppm (or g/ha),

Y is the efficacy when active compound B is applied at an application rate of n ppm (or g/ha),

E is the efficacy when the active compounds A and B are applied at application rates of m and n ppm (or g/ha), respectively, and

then

$$E = X + Y - \frac{X \cdot Y}{100}$$

The degree of efficacy, expressed in % is denoted. 0 % means an efficacy which corresponds to that of the control while an efficacy of 100 % means that no disease is observed.

If the actual fungicidal activity exceeds the calculated value, then the activity of the combination is superadditive, i.e. a synergistic effect exists. In this case, the efficacy which was actually observed must be greater than the value for the expected efficacy (E) calculated from the abovementioned formula.

A further way of demonstrating a synergistic effect is the method of Tammes (cf. "Isoboles, a graphic representation of synergism in pesticides" in Neth. J. Plant Path., 1964, 70, 73-80).

The invention is illustrated by the following example. However the invention is not limited to the example.

Example A: in vivo preventive test on *Alternaria* test (tomatoes)

| | | |
|---|---|---|
| Solvent: | 24.5 | parts by weight of acetone |
| | 24.5 | parts by weight of dimethylacetamide |
| Emulsifier: | 1 | part by weight of alkylaryl polyglycol ether |

To produce a suitable preparation of active compound, 1 part by weight of active compound is mixed with the stated amounts of solvent and emulsifier, and the concentrate is diluted with water to the desired concentration.

To test for preventive activity, young plants are sprayed with the preparation of active compound at the stated rate of application. After the spray coating has dried on, the plants are inoculated with an aqueous spore suspension of ***Alternaria solani.*** The plants are then placed in an incubation cabinet at approximately 20 °C and a relative atmospheric humidity of 100%.

The test is evaluated 3 days after the inoculation. 0% means an efficacy which corresponds to that of the untreated control while an efficacy of 100% means that no disease is observed.

The table below clearly shows that the observed activity of the active compound combination according to the invention is greater than the calculated activity, i.e. a synergistic effect is present.

Table: in vivo preventive test on *Alternaria* test (tomatoes)

| Active compounds | | | Application rate of active compound in ppm a.i. | Efficacy in % found* | Efficacy in % calc. ** |
|---|---|---|---|---|---|
| (I-1) | 3-(difluoromethyl)-N-methoxy-1-methyl-N-[1-(2,4,6-trichlorophenyl)propan-2-yl]-1H-pyrazole-4-carboxamide | | 0.25<br>0.125 | 85<br>73 | |
| (B1-4) | 3-(4,4-difluoro-3,3-dimethyl-3,4-dihydroisoquinolin-1-yl)quinolone | | 0.5<br>0.25<br>0.125 | 15<br>0<br>0 | |
| (I-1) + (B1-4) | | 1:1 | 0.25 + 0.25 | 90 | 85 |
| (I-1) + (B1-4) | | 1:0.5 | 0.25 + 0.125 | 90 | 85 |
| (I-1) + (B1-4) | | 1:4 | 0.125 + 0.5 | 88 | 77 |
| * found = activity found<br>** calc. = activity calculated using Colby's formula | | | | | |

Example B: in vivo preventive *Septoria tritici* test (wheat)

| | | |
|---|---|---|
| Solvent: | 49 | parts by weight of N,N-dimethylacetamide |
| Emulsifier: | 1 | part by weight of alkylaryl polyglycol ether |

To produce a suitable preparation of active compound, 1 part by weight of active compound or active compound combination is mixed with the stated amounts of solvent and emulsifier, and the concentrate is diluted with water to the desired concentration.

To test for preventive activity, young plants are sprayed with the preparation of active compound or active compound combination at the stated rate of application.

After the spray coating has been dried, the plants are sprayed with a spore suspension of ***Septoria tritici.*** The plants remain for 48 hours in an incubation cabinet at approximately 20 °C and a relative atmospheric humidity of approximately 100% and afterwards for 60 hours at approximately 15 °C in a translucent incubation cabinet at a relative atmospheric humidity of approximately 100%.

The plants are placed in the greenhouse at a temperature of approximately 15 °C and a relative atmospheric humidity of approximately 80%.

The test is evaluated 21 days after the inoculation. 0% means an efficacy which corresponds to that of the untreated control, while an efficacy of 100% means that no disease is observed.

The table below clearly shows that the observed activity of the active compound combination according to the invention is greater than the calculated activity, i.e. a synergistic effect is present.

Table: in vivo preventive test on *Septoria tritici* test (wheat)

| Active compounds | | Application rate of active compound in ppm a.i. | Efficacy in % found* | Efficacy in % calc. ** |
|---|---|---|---|---|
| (I-1) | 3-(difluoromethyl)-N-methoxy-1-methyl-N-[1-(2,4,6-trichlorophenyl)propan-2-yl]-1H-pyrazole-4-carboxamide | 10<br>5 | 63<br>50 | |
| (B1-1) | 9-fluoro-2,2-dimethyl-5-(quinolin-3-yl)-2,3-dihydro-1,4-benzoxazepine | 50 | 25 | |

(continued)

| Active compounds | | Application rate of active compound in ppm a.i. | Efficacy in % | |
|---|---|---|---|---|
| | | | found* | calc. ** |
| (B1-2) 2-{2-ffuoro-6-[(8-fluoro-2-methylquinolin-3-yl)oxy] phenyl}propan-2-ol | | 50 | 0 | |
| (B1-3) 2-{2-[(7,8-difluoro-2-methylquinolin-3-yl)oxcy]-6-fluorophenyl}propan-2-ol | | 50 | 25 | |
| (B1-4) 3-(4,4-difluoro-3,3-dimethyl-3,4-dihydroisoquinolin-1-yl)quinolone | | 60 | 13 | |
| (I-1) + (B1-1) | 1:4 | 10+50 | 100 | 72 |
| (I-1) + (B1-1) | 1:10 | 5+50 | 100 | 63 |
| (I-1) + (B1-2) | 1:4 | 10+50 | 100 | 63 |
| (I-1) + (B1-2) | 1:10 | 5+50 | 100 | 50 |
| (I-1) + (B1-3) | 1:4 | 10+50 | 100 | 72 |
| (I-1) + (B1-3) | 1:10 | 5+50 | 100 | 63 |
| (I-1) + (B1-4) | 1:6 | 10+60 | 100 | 68 |
| (I-1) + (B1-4) | 1:12 | 5+60 | 100 | 57 |
| * found = activity found<br>** calc. = activity calculated using Colby's formula | | | | |

## Claims

**1.** A composition comprising

(A) at least one compound of formula (I)

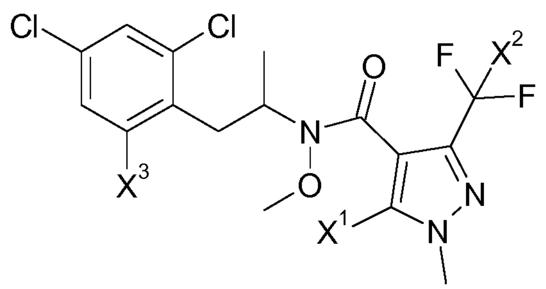

(I),

in which $X^1$, $X^2$ and $X^3$ independently from each other represent a hydrogen atom, a fluorine atom or a chlorine atom,
or agrochemically acceptable salts or isomers or enantiomers or tautomers or N-oxides thereof, and
(B) at least one compound selected from the group consisting of

Compound (B1-1)

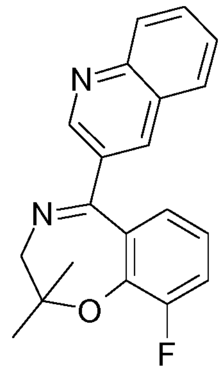

(B1-1)

(9-fluoro-2,2-dimethyl-5-(quinolin-3-yl)-2,3-dihydro-1,4-benzoxazepine),

Compound (B1-2)

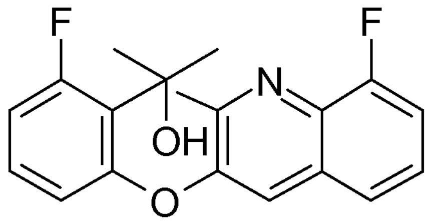

(B1-2)

(2-{2-fluoro-6-[(8-fluoro-2-methylquinolin-3-yl)oxy]phenyl}propan-2-ol),

Compound (B1-3)

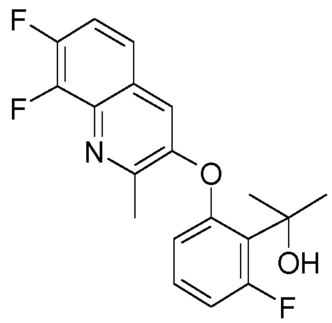

(B1-3)

(2-{2-[(7,8-difluoro-2-methylquinolin-3-yl)oxy]-6-fluorophenyl}propan-2-ol),

Compound (B1-4)

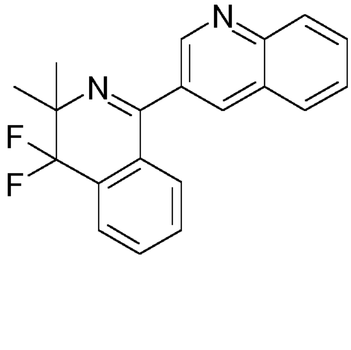

(B1-4)

(3-(4,4-difluoro-3,3-dimethyl-3,4-dihydroisoquinolin-1-yl)quinolone).

2. A composition according to claim 1 wherein (A) is a compound according to formula (I), wherein $X^1$ represents a hydrogen atom, $X^2$ represents a hydrogen atom and $X^3$ represents a chlorine atom.

3. A composition according to claim 1 wherein (A) is a compound according to formula (I-1)

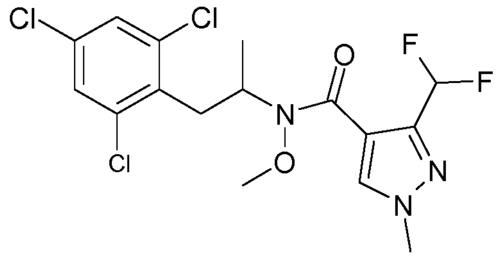

(I-1)

(3-(difluoromethyl)-N-methoxy-1-methyl-N-[1-(2,4,6-trichlorophenyl)propan-2-yl]-1H-pyrazole-4-carboxamide).

4. A composition according to claim 1 wherein (A) is a compound according to formula (I-2)

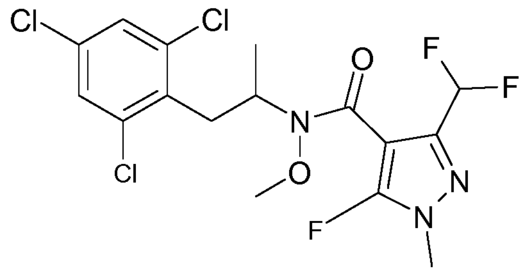

(I-2)

(3-(difluoromethyl)-5-fluoro-N-methoxy-1-methyl-N-[1-(2,4,6-trichlorophenyl)propan-2-yl]-1H-pyrazole-4-carboxamide).

5. A composition according to any of claims 1 to 4 and further comprising auxiliaries, solvents, carriers, surfactants and/or extenders.

6. Method for controlling phytopathogenic fungi, **characterized in that** compositions according to any of Claims 1 to 5 are applied to the phytopathogenic fungi and/or their habitat.

7. Use of a composition according to any of Claims 1 to 5 for the control of phytopathogenic fungi.

8. Process for producing a composition for controlling phytopathogenic fungi, **characterized in that** compositions according to any of Claims 1 to 5 are mixed with auxiliaries, solvents, carriers, surfactants and/or extenders.

9. Use according to Claim 7 wherein transgenic plants are treated.

10. Use according to Claim 7 wherein seed and seed of transgenic plants are treated.

**Patentansprüche**

1. Zusammensetzung, umfassend

(A) mindestens eine Verbindung der Formel (I)

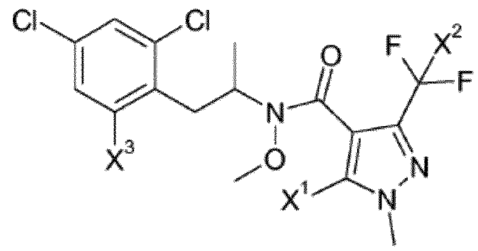

(I),

in der $X^1$, $X^2$ und $X^3$ unabhängig voneinander ein Wasserstoffatom, ein Fluoratom oder ein Chloratom bedeuten, oder agrarchemisch unbedenkliche Salze oder Isomere oder Enantiomere oder Tautomere oder N-Oxide davon, sowie

(B) mindestens eine Verbindung, ausgewählt aus der Gruppe bestehend aus

Verbindung (B1-1)

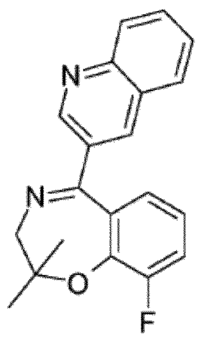

(B1-1)

(9-Fluor-2,2-dimethyl-5-(chinolin-3-yl)-2,3-dihydro-1,4-benzoxazepin),

Verbindung (B1-2)

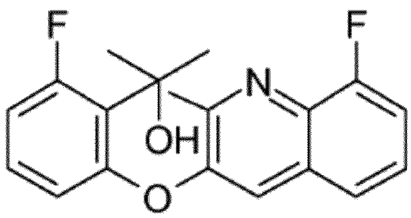

(B1-2)

(2-{2-Fluor-6-[(8-fluor-2-methylchinolin-3-yl)oxy]phenyl}propan-2-ol),

Verbindung (B1-3)

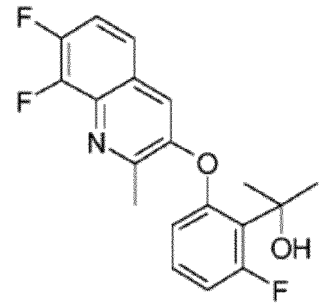

(B1-3)

(2-{2-[(7,8-Difluor-2-methylchinolin-3-yl)oxy]-6-fluorphenyl}propan-2-ol),

Verbindung (B1-4)

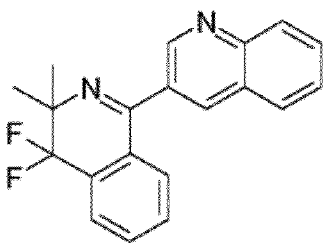

(B1-4)

(3-(4,4-Difluor-3,3-dimethyl-3,4-dihydroisochinolin-1-yl)chinolon).

2. Zusammensetzung nach Anspruch 1, wobei (A) eine Verbindung der Formel (I) ist, wobei $X^1$ ein Wasserstoffatom bedeutet, $X^2$ ein Wasserstoffatom bedeutet und $X^3$ ein Chloratom bedeutet.

3. Zusammensetzung nach Anspruch 1, wobei (A) eine Verbindung der Formel (I-1)

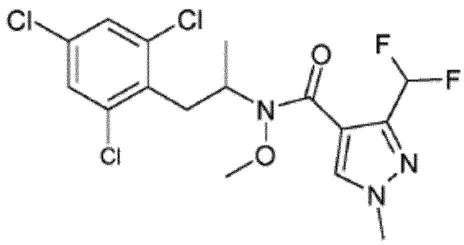

(I-1)

(3-(Difluormethyl)-N-methoxy-1-methyl-N-[1-(2,4,6-trichlorphenyl)propan-2-yl]-1H-pyrazol-4-carboxamid) bedeutet.

4. Zusammensetzung nach Anspruch 1, wobei (A) eine Verbindung nach Formel (I-2)

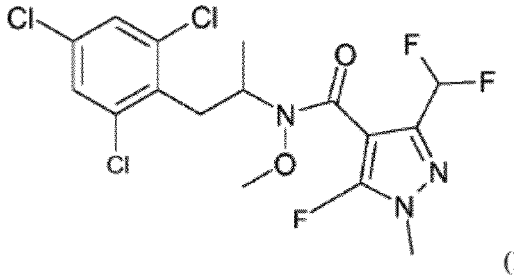

(I-2)

(3-(Difluormethyl)-5-fluor-N-methoxy-1-methyl-N-[1-(2,4,6-trichlorphenyl)propan-2-yl]-1H-pyrazol-4-carboxamid) bedeutet.

5. Zusammensetzung nach einem der Ansprüche 1 bis 4, weiterhin umfassend Hilfsstoffe, Lösungsmittel, Träger, Tenside und/oder Streckmittel.

6. Verfahren zum Bekämpfen von phytopathogenen Pilzen, **dadurch gekennzeichnet, dass** man Zusammensetzungen nach einem der Ansprüche 1 bis 5 auf die phytopathogenen Pilze und/oder ihren Lebensraum ausbringt.

7. Verwendung einer Zusammensetzung nach einem der Ansprüche 1 bis 5 für die Bekämpfung von phytopathogenen Pilzen.

8. Verfahren zur Herstellung einer Zusammensetzung für die Bekämpfung von phytopathogenen Pilzen, **dadurch gekennzeichnet, dass** man Zusammensetzungen nach einem der Ansprüche 1 bis 5 mit Hilfsstoffen, Lösungsmitteln, Trägern, Tensiden und/oder Streckmitteln mischt.

9. Verwendung nach Anspruch 7, wobei transgene Pflanzen behandelt werden.

10. Verwendung nach Anspruch 7, wobei Saatgut und Saatgut von transgenen Pflanzen behandelt werden.

**Revendications**

1. Composition comprenant

(A) au moins un composé d formule (I)

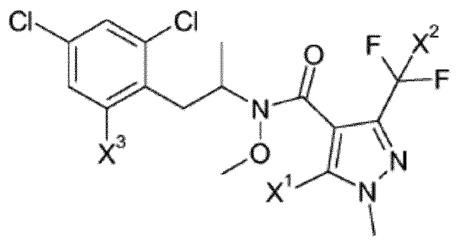

(I),

où $X^1$, $X^2$ et $X^3$, indépendamment les uns des autres, représentent un atome d'hydrogène, un atome de fluor ou un atome de chlore,
ou des sels ou isomères ou énantiomères ou tautomères ou N-oxydes acceptables sur le plan agrochimique de celui-ci, et
(B) au moins un composé choisi dans le groupe constitué par :

Composé (B1-1)

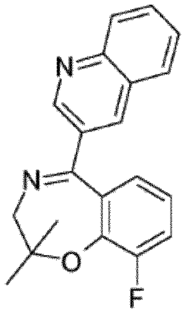

(B1-1)

(9-fluoro-2,2-diméthyl-5-(quinoléin-3-yl)-2,3-dihydro-1,4-benzoxazépine),

Composé (B1-2)

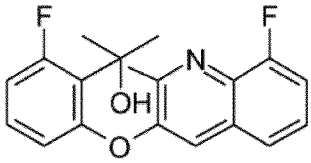

(B1-2)

(2-{2-fluoro-6-[(8-fluoro-2-méthylquinoléin-3-yl)oxy]-phényl}propan-2-ol),

Composé (B1-3)

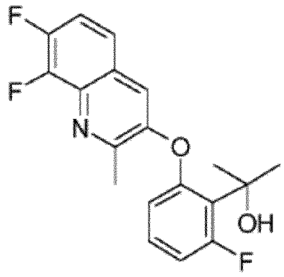

(B1-3)

(2-{2-[(7,8-fluoro-2-méthylquinoléin-3-yl)oxy]6-fluoro-phényl}propan-2-ol),

Composé (B1-4)

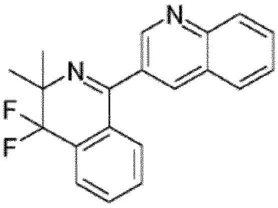

(B1-4)

(3-(4,4-difluoro-3,3-diméthyl-3,4-dihydroisoquinoléin-1-yl)quinolone).

**2.** Composition selon la revendication 1, dans laquelle (A) est un composé répondant à la formule (I), où $X^1$ représente un atome d'hydrogène, $X^2$ représente un atome d'hydrogène et $X^3$ représente un atome de chlore.

**3.** Composition selon la revendication 1, dans laquelle (A) est un composé répondant à la formule (I-1)

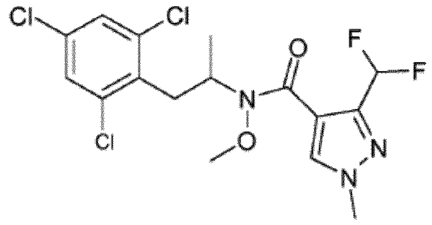

(I-1)

(3-(difluorométhyl)-N-méthoxy-1-méthyl-N-[1-(2,4,6-trichlorophényl)propan-2-yl]-1H-pyrazole-4-carboxamide).

4. Composition selon la revendication 1, dans laquelle (A) est un composé répondant à la formule (I-2)

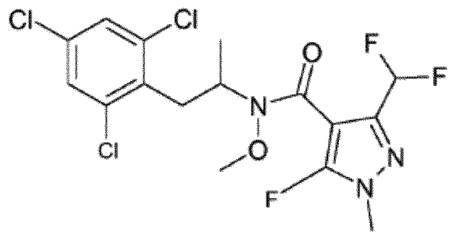

(I-2)

(3-(difluorométhyl)-5-fluoro-N-méthoxy-1-méthyl-N-[1-(2,4,6-trichlorophényl)propan-2-yl]-1H-pyrazole-4-carboxamide).

5. Composition selon l'une quelconque des revendications 1 à 4, et comprenant en outre des auxiliaires, des solvants, des véhicules, des agents tensioactifs et/ou des matières de charge.

6. Méthode de contrôle de champignons phytopathogènes, **caractérisée en ce que** des compositions selon l'une quelconque des revendications 1 à 5 sont appliquées aux champignons phytopathogènes et/ou à leur habitat.

7. Utilisation d'une composition selon l'une quelconque des revendications 1 à 5, pour le contrôle de champignons phytopathogènes.

8. Procédé de production d'une composition destinée au contrôle de champignons phytopathogènes, **caractérisé en ce que** des compositions selon l'une quelconque des revendications 1 à 5 sont mélangées avec des auxiliaires, des solvants, des véhicules, des agents tensioactifs et/ou des matières de charge.

9. Utilisation selon la revendication 7, dans laquelle des plantes transgéniques sont traitées.

10. Utilisation selon la revendication 7, dans laquelle des semences et des semences de plantes transgéniques sont traitées.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2010063700 A
- WO 2013127764 A
- WO 2013167651 A
- WO 2012021250 A
- WO 2012069652 A
- WO 2012146125 A
- WO 2013007550 A
- WO 2013011010 A
- WO 2012041874 A
- WO 2014016279 A
- US 4272417 A
- US 4245432 A
- US 4808430 A
- US 5876739 A
- US 20030176428 A1
- WO 2002080675 A
- WO 2002028186 A
- US 765491 A
- US 11765494 B
- US 10926819 B
- US 10782020 B
- US 12032479 B
- US 10783417 B
- US 10782096 B
- US 11657964 B
- US 12192904 B
- US 11396808 B
- US 12166253 B
- US 12166239 B
- US 12166124 B
- US 12166209 B
- US 11762886 B
- US 12364335 B
- US 11763947 B
- US 12252453 B
- US 12209354 B
- US 12491396 B
- US 12497221 B
- US 12644632 B
- US 12646004 B
- US 12701058 B
- US 12718059 B
- US 12721595 B
- US 12638591 B
- WO 9205251 A
- WO 9509910 A
- WO 9827806 A
- WO 05002324 A
- WO 06021972 A
- US 6229072 B
- WO 8910396 A
- WO 9102069 A
- WO 0166704 A
- EP 0837944 A
- WO 0066746 A
- WO 0066747 A
- WO 0226995 A
- US 5776760 A
- US 5463175 A
- WO 02036782 A
- WO 03092360 A
- WO 2005012515 A
- WO 2007024782 A
- WO 01024615 A
- WO 03013226 A
- US 517991 A
- US 10739610 B
- US 12139408 B
- US 12352532 B
- US 11312866 B
- US 11315678 B
- US 12421292 B
- US 11400598 B
- US 11651752 B
- US 11681285 B
- US 11605824 B
- US 12468205 B
- US 11760570 B
- US 11762526 B
- US 11769327 B
- US 11769255 B
- US 11943801 B
- US 12362774 B
- US 588811 A
- US 11185342 B
- US 12364724 B
- US 11185560 B
- US 12423926 B
- US 760602 A
- US 5561236 A
- US 5648477 A
- US 5646024 A
- US 5273894 A
- US 5637489 A
- US 5276268 A
- US 5739082 A
- US 5908810 A
- US 7112665 B

- WO 9638567 A
- WO 9924585 A
- WO 9924586 A
- WO 09144079 A
- WO 02046387 A
- US 6768044 B
- WO 9934008 A
- WO 0236787 A
- WO 04024928 A
- WO 2007103567 A
- WO 2008150473 A
- US 5605011 A
- US 5378824 A
- US 5141870 A
- US 5013659 A
- US 5767361 A
- US 5731180 A
- US 5304732 A
- US 4761373 A
- US 5331107 A
- US 5928937 A
- WO 9633270 A
- WO 2004040012 A
- WO 2004106529 A
- WO 2005020673 A
- WO 2005093093 A
- WO 2006007373 A
- WO 2006015376 A
- WO 2006024351 A
- WO 2006060634 A
- US 61288958 A
- US 5084082 A
- WO 9741218 A
- US 5773702 A
- WO 99057965 A
- US 5198599 A
- WO 01065922 A
- EP 1999141 A
- WO 2007107302 A
- US 249016 A
- US 214022 A
- EP 2300618 A
- WO 2007027777 A
- WO 9421795 A
- US 61126083 A
- US 61195019 B
- WO 2007080126 A
- WO 2006129204 A
- WO 2007074405 A
- WO 2007080127 A
- WO 2007035650 A
- WO 0004173 A
- WO 2006045633 A
- EP 1807519 A
- EP 2018431 A
- WO 2004090140 A
- EP 1794306 A
- WO 2006133827 A
- WO 2007107326 A
- EP 1999263 A
- EP 0571427 A
- WO 9504826 A
- EP 0719338 A
- WO 9615248 A
- WO 9619581 A
- WO 9627674 A
- WO 9711188 A
- WO 9726362 A
- WO 9732985 A
- WO 9742328 A
- WO 9744472 A
- WO 9745545 A
- WO 9827212 A
- WO 9840503 A
- WO 9958688 A
- WO 9958690 A
- WO 9958654 A
- WO 0008184 A
- WO 0008185 A
- WO 0008175 A
- WO 0028052 A
- WO 0077229 A
- WO 0112782 A
- WO 0112826 A
- WO 02101059 A
- WO 03071860 A
- WO 04056999 A
- WO 05030942 A
- WO 2005030941 A
- WO 2005095632 A
- WO 2005095617 A
- WO 2005095619 A
- WO 2005095618 A
- WO 2005123927 A
- WO 2006018319 A
- WO 2006103107 A
- WO 2006108702 A
- WO 2007009823 A
- WO 0022140 A
- WO 2006063862 A
- WO 2006072603 A
- WO 02034923 A
- WO 2008017518 A
- WO 2008080630 A
- WO 2008080631 A
- EP 07090007 A
- WO 2008090008 A
- WO 0114569 A
- WO 0279410 A
- WO 0333540 A
- WO 2004078983 A
- WO 0119975 A
- WO 9526407 A
- WO 9634968 A
- WO 9820145 A
- WO 9912950 A

- WO 9966050 A
- WO 9953072 A
- US 6734341 B
- WO 0011192 A
- WO 9822604 A
- WO 9832326 A
- WO 0198509 A
- WO 2005002359 A
- US 5824790 A
- US 6013861 A
- WO 9404693 A
- WO 9409144 A
- WO 9411520 A
- WO 9535026 A
- WO 9720936 A
- WO 2010012796 A
- WO 2010003701 A
- EP 0663956 A
- WO 9601904 A
- WO 9621023 A
- WO 9839460 A
- WO 9924593 A
- WO 9531553 A
- US 2002031826 A
- US 6284479 B
- US 5712107 A
- WO 9747806 A
- WO 9747807 A
- WO 9747808 A
- WO 0014249 A
- WO 0073422 A
- WO 0047727 A
- EP 06077301 A
- US 5908975 A
- EP 0728213 A
- WO 2006032538 A
- WO 2007039314 A
- WO 2007039315 A
- WO 2007039316 A
- JP 2006304779 A
- WO 2005012529 A
- US 020360 A
- US 61054026 B
- WO 9800549 A
- WO 2004053219 A
- WO 0117333 A
- WO 0245485 A
- WO 2005017157 A
- WO 2009143995 A
- WO 2006136351 A
- US 5969169 A
- US 5840946 A
- US 6323392 B
- US 6063947 A
- US 6270828 B
- US 6169190 B
- US 5965755 A
- US 5434283 A
- US 668303 A
- US 61135230 A
- WO 2009068313 A
- WO 2010006732 A
- WO 2010121818 A
- WO 2010145846 A
- WO 2002040677 A
- WO 06128569 A
- WO 06128570 A
- US 2002120964 A
- WO 02034946 A
- WO 10117737 A
- WO 10117735 A
- WO 05103266 A
- US 2005216969 A
- US 2007143876 A
- WO 06098952 A
- US 2006230473 A
- WO 2002027004 A
- WO 11075593 A
- WO 11075595 A
- WO 10077816 A
- US 2006162007 A
- WO 04053062 A
- US 2003126634 A
- WO 10080829 A
- WO 2005074671 A
- US 2009217423 A
- WO 06128573 A
- US 20100024077 A
- WO 06128571 A
- WO 06128572 A
- US 2006130175 A
- WO 04039986 A
- US 2007067868 A
- WO 05054479 A
- WO 05054480 A
- WO 012033794 A
- WO 11022469 A
- WO 2012075426 A
- WO 2012075429 A
- US 2006070139 A
- WO 09100188 A
- WO 11066384 A
- WO 11066360 A
- US 2009137395 A
- WO 08112019 A
- US 2008312082 A
- WO 08054747 A
- US 20090210970 A
- WO 09103049 A
- US 20100184079 A
- WO 08002872 A
- WO 07091277 A
- US 2006059581 A
- WO 98044140 A
- WO 2011063413 A
- US 2005086719 A

- US 2005188434 A
- WO 08151780 A
- US 2010050282 A
- WO 07017186 A
- WO 10076212 A
- US 2004172669 A
- WO 04074492 A
- US 2008064032 A
- WO 06108674 A
- US 2008320616 A
- WO 06108675 A
- US 2008196127 A
- WO 03013224 A
- US 2003097687 A
- US 6468747 B
- WO 00026345 A
- WO 2000026345 A
- US 20082289060 A
- WO 00026356 A
- US 2007028322 A
- WO 05061720 A
- US 2009300784 A
- WO 07142840 A
- US 2008167456 A
- WO 05103301 A
- US 2004250317 A
- WO 02100163 A
- US 2002102582 A
- WO 04011601 A
- US 2006095986 A
- WO 11062904 A
- WO 09111263 A
- US 20110138504 A
- US 2009130071 A
- WO 09064652 A
- US 20100080887 A
- WO 10037016 A
- WO 11034704 A
- WO 2012051199 A
- WO 10024976 A
- US 20110067141 A
- WO 09102873 A
- US 2008028482 A
- WO 05059103 A
- WO 04072235 A
- US 2006059590 A
- WO 2011153186 A
- WO 2012134808 A
- WO 07140256 A
- US 2008260932 A
- US 2006282915 A
- WO 06130436 A
- WO 01031042 A
- WO 01041558 A
- US 2003188347 A
- US 2007292854 A
- WO 08114282 A
- WO 02036831 A
- US 2008070260 A
- WO 2012082548 A
- WO 0244407 A
- US 2009265817 A
- US 2001029014 A
- WO 01051654 A
- US 2010077501 A
- WO 08122406 A
- WO 06128568 A
- US 2005039226 A
- WO 04099447 A
- WO 03052073 A
- WO 11084632 A
- WO 11084621 A
- WO 2011063413 A2
- WO 2011066360 A1
- WO 2011066384 A1
- WO 2011075593 A1
- WO 2011075595 A1
- WO 2011084621 A1
- WO 2011084632 A1
- WO 2011153186 A1
- WO 2012033794 A2
- WO 2012051199 A2
- WO 2012075426 A1
- WO 2012075429 A1
- WO 2012082548 A2
- WO 2012071039 A1
- US 2012131692 A
- WO 2012075426 A2
- WO 2012075429 A2
- WO 2012134808 A1
- WO 2013003558 A1
- WO 2013010094 A1
- WO 2013012775 A1
- WO 2013014241 A1
- WO 2013016516 A1
- WO 2013016527 A1
- WO 2013025400 A1
- WO 2013112525 A2
- WO 2013112527 A1

**Non-patent literature cited in the description**

- *Czech J. Food Sci.,* 2000, vol. 21 (3), 91-96
- *dvanced Techniques in Soil Microbiology,* 2007, vol. 11, 319-341
- *Applied Soil Ecology,* 2000, vol. 15, 169-182
- **R. WEGLER.** Chemie der Pflanzenschutz- und Schadlingsbekampfungsmittel'' [Chemistry of the Crop Protection Compositions and Pesticides. Springer Verlag, 1970, vol. 2, 401-412

- *Science,* 1983, vol. 221, 370-371
- *Curr. Tropics Plant Physiol.,* 1992, vol. 7, 139-145
- *Science,* 1986, vol. 233, 478-481
- *J. Biol. Chem.,* 1988, vol. 263, 4280-4289
- **TRANEL ; WRIGHT.** *Weed Science,* 2002, vol. 50, 700-712
- **CRICKMORE et al.** *Microbiology and Molecular Biology,* 1998, vol. 62, 807-813
- *Nat. Biotechnol.,* 2001, vol. 19, 668-72
- *Applied Environm. Microbiol.,* 2006, vol. 71, 1765-1774
- **COL-BY, S.R.** Calculating Synergistic and Antagonistic Responses of Herbicide Combinations. *Weeds,* 1967, vol. 15, 20-22
- Isoboles, a graphic representation of synergism in pesticides. *Neth. J. Plant Path.,* 1964, vol. 70, 73-80